# EUROPEAN PATENT SPECIFICATION

(11) **EP 4 262 689 B1**
(45) Date of publication and mention of the grant of the patent: **08.04.2026**
(21) Application number: 21835322.5
(22) Date of filing: 16.12.2021
(51) Int. Cl.: A61K 8/31, A61K 8/41, A61K 8/44, A61K 8/46, A61K 8/49, A61K 8/60, A61K 8/73, A61K 8/92, A61Q 5/10

(54) **COMPOSITION COMPRISING A PARTICULAR OXIDATION DYEING BASE, AT LEAST ONE GUAR GUM AND AT LEAST ONE FATTY SUBSTANCE**
ZUSAMMENSETZUNG MIT EINER BESTIMMTEN OXIDATIONSFÄRBEBASIS, MINDESTENS EINEM GUARGUMMI UND MINDESTENS EINER FETTSUBSTANZ
COMPOSITION COMPRENANT UNE BASE DE COLORATION D'OXYDATION PARTICULIÈRE, AU MOINS UNE GOMME DE GUAR ET AU MOINS UNE SUBSTANCE GRASSE

(30) Priority: 17.12.2020 FR 2013504
(43) Date of publication of application: 25.10.2023
(73) Proprietor: L'OREAL, 75008 Paris (FR)
(72) Inventor: PAILLARD-BRUNET, Maxime, 93400 Saint-Ouen (FR); BLANC, Julie, 93400 Saint-Ouen (FR)
(74) Representative: L'Oreal
(86) International application number: PCT/EP2021/086275
(87) International publication number: WO 2022/129386

(56) References cited:
- CA-A1- 2 576 189
- FR-A1- 3 026 007
- US-A1- 2015 202 125

## Description

The invention relates to a composition comprising at least one particular dyeing base, at least one guar gum, and at least one fatty substance, the total content of fatty substance being greater than or equal to 30% by weight, relative to the total weight of the composition.

The invention also relates to a process for dyeing keratin fibres, in particular the hair, using this composition.

Finally, the invention relates to the use of such a composition for dyeing keratin fibres, and in particular the hair.

Many people have sought for a long time to modify the colour of their hair and in particular to mask their grey hair.

It is known practice to dye keratin fibres, in particular human keratin fibres such as the hair, to obtain permanent colourings with dyeing compositions containing oxidation dye precursors, in particular oxidation bases, such as ortho- or para-phenylenediamines, ortho- or para-aminophenols, or heterocyclic compounds such as pyrazoles, pyrazolinones or pyrazolo-pyridines. These oxidation bases are colourless or weakly coloured compounds which, when combined with oxidizing products, can give rise to coloured compounds via a process of oxidative condensation.

It is also possible to vary the shades obtained with these oxidation bases by combining them with couplers or colour modifiers. The variety of molecules used as oxidation bases and couplers allows a wide range of colours to be obtained. However, the use of these dye compositions may have a certain number of drawbacks.

Specifically, after application to keratin fibres, the dyeing power obtained may not be entirely satisfactory, or may even be weak, and lead to a restricted range of colours.

The colourings may also be insufficiently persistent with respect to external agents such as light, shampoo or perspiration, and may also be too selective, i.e. the difference in colouring is too great along the same keratin fibre that is differently sensitized between its end and its root.

By way of example, 2-(methoxymethyl)benzene-1,4-diamine, which is described in the document CA2576189 and which is known to be a dye precursor of interest, does not make it possible to achieve entirely satisfactory dyeing properties, in particular in terms of selectivity. Specifically, it does not make it possible to obtain a colour result which is uniform from the root to the end. In addition, it does not make it possible to ensure good coverage of the keratin fibres, and in particular of depigmented keratin fibres, such as grey hair. Furthermore, its dyeing power proves to be too limited, thus leading to a restricted dyeing range.

French patent application FR 3026007 A1 also discloses the use of 2-(methoxymethyl)benzene-1,4-diamine in a hair dye composition comprising more than 30% by weight of fatty substances. Hair dye compositions including a dye precursor, guar gum such as hydroxypropyl guar and more than 30% by weight of fatty substances are described in the US patent application US 2015202125 A1.

Thus, there is a real need to provide a composition for dyeing keratin fibres, in particular human keratin fibres such as the hair, which does not have the drawbacks mentioned above, i.e. which is capable of resulting in a good colour build-up and good colour strength while at the same time having a low selectivity and a good fastness, and which is capable of resulting in good dyeing performance, even after a period of storage.

These aims and others are achieved by the present invention, one subject of which is thus a composition comprising:
- 2-(methoxymethyl)benzene-1,4-diamine of formula (I), its addition salts, its solvates and/or the solvates of its salts: 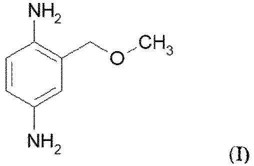
- at least one guar gum,
- at least one fatty substance, the total content of fatty substance being greater than or equal to 30% by weight, relative to the total weight of the composition.

According to a preferred embodiment, the composition according to the invention is a composition for dyeing keratin fibres, in particular the hair.

The composition according to the invention may especially lead to chromatic, powerful, intense and sparingly selective colourings, i.e. colourings that are uniform along the length of the fibre. It also allows various shades to be achieved in a very wide range of colours. Furthermore, it enables a good colour build-up.

This composition also gives particularly good coverage of depigmented keratin fibres such as grey hair.

A subject of the invention is also a kit comprising, in a first compartment, a composition as defined above and, in a second compartment, an oxidizing composition comprising at least one chemical oxidizing agent.

According to the invention, the term "chemical oxidizing agent" is intended to mean an oxidizing agent other than atmospheric oxygen.

Other subjects, characteristics, aspects and advantages of the invention will become even more clearly apparent on reading the description and the examples which follow.

In that which follows, and unless otherwise indicated, the limits of a range of values are included in this range, in particular in the expressions "of between" and "ranging from ... to ...".

Moreover, the expression "at least one" used in the present description is equivalent to the expression "one or more".

### Oxidation bases

The composition according to the invention comprises at least one particular oxidation base.

The composition according to the invention comprises at least 2-(methoxymethyl)benzene-1,4-diamine of formula (I), its addition salts, its solvates and/or the solvates of its salts.

The addition salts of the compound of formula (I) present in the composition according to the invention are chosen especially from the addition salts with an acid, such as the hydrochlorides, hydrobromides, sulfates, citrates, succinates, tartrates, lactates, tosylates, benzenesulfonates, phosphates and acetates, and the addition salts with a base such as sodium hydroxide, potassium hydroxide, aqueous ammonia, amines or alkanolamines.

Moreover, the solvates of the compound of formula (I) more particularly represent the hydrates of said compound and/or the combination of said compound with a linear or branched C1 to C4 alcohol such as methanol, ethanol, isopropanol or n-propanol. Preferably, the solvates are hydrates.

The total content of 2-(methoxymethyl)benzene-1,4-diamine of formula (I), of its addition salts, of its solvates and/or of the solvates of its salts preferably ranges from 0.001% to 20% by weight, more preferentially from 0.005% to 15% by weight, better still from 0.01% to 10% by weight, even better still from 0.05% to 5% by weight, better yet from 0.1% to 3% by weight, relative to the total weight of the composition.

The composition according to the invention may optionally further comprise one or more additional oxidation bases different from the compound of formula (I), chosen from para-phenylenediamines, bis(phenyl)alkylenediamines, para-aminophenols, ortho-aminophenols and heterocyclic bases, their addition salts and their solvates.

Among the para-phenylenediamines different from the compound of formula (I), mention may be made, for example, of para-phenylenediamine, para-toluenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene and 3-hydroxy-1-(4'-aminophenyl)pyrrolidine, their addition salts with an acid and their solvates.

Among the bis(phenyl)alkylenediamines, mention may for example be made of N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane, their addition salts with an acid and their solvates.

Among the para-aminophenols, mention may for example be made of para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, their addition salts with an acid and their solvates.

Among the ortho-aminophenols, examples that may be mentioned include 2-aminophenol, 2-amino-5-methylphenol, 2-amino-6-methylphenol and 5-acetamido-2-aminophenol, their addition salts with an acid and their solvates.

Among the heterocyclic bases, examples that may be mentioned include pyridine derivatives, pyrimidine derivatives and pyrazole derivatives.

Among the pyridine derivatives, mention may be made of the compounds described, for example, in patents GB 1 026 978 and GB 1 153 196, such as 2,5-diaminopyridine, 2-(4-methoxyphenyl)amino-3-aminopyridine, 3,4-diaminopyridine, their addition salts with an acid and their solvates.

Among the pyrimidine derivatives, mention may be made of the compounds described, for example, in patents DE 2 359 399; JP 88-169 571; JP 05-63124; EP 0 770 375 or patent application WO 96/15765, for instance 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine and 2,5,6-triaminopyrimidine, and pyrazolopyrimidine derivatives such as those mentioned in patent application FR-A-2 750 048, and among which mention may be made of pyrazolo[1,5-a]pyrimidine-3,7-diamine, 2,5-dimethylpyrazolo[1,5-a]pyrimidine-3,7-diamine, pyrazolo[1,5-a]pyrimidine-3,5-diamine, 2,7-dimethylpyrazolo[1,5-a]pyrimidine-3,5-diamine, 3-aminopyrazolo[1,5-a]pyrimidin-7-ol, 3-aminopyrazolo[1,5-a]pyrimidin-5-ol, 2-(3-aminopyrazolo[1,5-a]pyrimidin-7-ylamino)ethanol, 2-(7-aminopyrazolo[1,5-a]pyrimidin-3-ylamino)ethanol, 2-[(3-aminopyrazolo[1,5-a]pyrimidin-7-yl)(2-hydroxyethyl)amino]ethanol, 2-[(7-aminopyrazolo[1,5-a]pyrimidin-3-yl)(2-hydroxyethyl)amino]ethanol, 5,6-dimethylpyrazolo[1,5-a]pyrimidine-3,7-diamine, 2,6-dimethylpyrazolo[1,5-a]pyrimidine-3,7-diamine, 2,5-N7,N7-tetramethylpyrazolo[1,5-a]pyrimidine-3,7-diamine and 3-amino-5-methyl-7-imidazolylpropylaminopyrazolo[1,5-a]pyrimidine, their addition salts and their solvates.

Among the pyrazole derivatives, mention may be made of diaminopyrazole bases, especially the compounds described in patents DE 3843892 and DE 4133957 and patent applications WO 94/08969, WO 94/08970, FR-A-2 733 749 and DE 195 43 988, for instance 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the corresponding addition salts. It is also possible to use 4,5-diamino-1-(β-methoxyethyl)pyrazole, the addition salts thereof and the solvates thereof.

The addition salts of the oxidation bases present in the composition according to the invention are chosen especially from the addition salts with an acid, such as the hydrochlorides, hydrobromides, sulfates, citrates, succinates, tartrates, lactates, tosylates, benzenesulfonates, methanesulfonates, phosphates and acetates, and the addition salts with a base such as sodium hydroxide, potassium hydroxide, aqueous ammonia, amines or alkanolamines.

Moreover, the solvates of the additional oxidation bases more particularly represent the hydrates of said oxidation bases and/or the combination of said oxidation bases with a linear or branched C₁ to C₄ alcohol such as methanol, ethanol, isopropanol or n-propanol. Preferably, the solvates are hydrates.

In a particular embodiment, the composition according to the invention is free from oxidation bases chosen from para-phenylenediamine, para-toluenediamine, their addition salts, their solvates and the solvates of their salts.

When the composition according to the invention comprises one or more additional oxidation bases, the total content of the additional oxidation base(s), different from 2-(methoxymethyl)benzene-1,4-diamine of formula (I), its addition salts, its solvates and the solvates of its salts, present in the composition according to the invention, preferably ranges from 0.001% to 20% by weight, more preferentially from 0.005% to 15% by weight, better still from 0.01% to 10% by weight, even better still from 0.05% to 5% by weight, better yet from 0.1% to 3% by weight, relative to the total weight of the composition.

### Guar gums

The composition according to the present invention comprises one or more guar gums.

Guar gums usable according to the invention can be nonionic or cationic or anionic or amphoteric; preferably the guar gums according to the invention can be nonionic or cationic.

According to the invention, use may be made of chemically modified or unmodified nonionic guar gums.

The unmodified nonionic guar gums are, for example, the products sold under the name Vidogum GH 175 by the company Unipectine and under the names Meypro-Guar 50 and Jaguar C by the company Rhodia Chimie.

The modified nonionic guar gums that may be used according to the invention are preferably modified with C1-C6 hydroxyalkyl groups.

Among the hydroxyalkyl groups, mention may be made, by way of example, of hydroxymethyl, hydroxyethyl, hydroxypropyl and hydroxybutyl groups.

These hydroxyalkylated guar gums are well known from the prior art and may be prepared, for example, by reacting corresponding alkene oxides, for instance, propylene oxides, with the guar gum so as to obtain a guar gum modified with hydroxypropyl groups.

The degree of hydroxyalkylation, which corresponds to the number of alkylene oxide molecules consumed by the number of free hydroxyl functions present on the guar gum, preferably ranges from 0.4 to 1.2.

Such nonionic guar gums optionally modified with hydroxyalkyl groups are sold, for example, under the trade names Jaguar HP8, Jaguar HP60 and Jaguar HP120, Jaguar DC 293 and Jaguar HP 105 by the company Meyhall, or under the name Galactasol 4H4FD2 by the company Aqualon.

Also suitable for use are nonionic guar gums modified with hydroxyalkyl groups, more especially hydroxypropyl groups, modified with groups comprising at least one C₆-C₃₀ fatty chain. Examples of such compounds that may be mentioned include, inter alia, the product Esaflor HM 22^{®} (C₂₂ alkyl chain) sold by the company Lamberti, and the products RE210-18^{®} (C₁₄ alkyl chain) and RE205-1^{®} (C₂₀ alkyl chain) sold by the company Rhône-Poulenc.

The cationic guar gums that may more particularly be used according to the invention are guar gums comprising trialkylammonium cationic groups. Preferably, 2% to 30% and more preferentially still 5% to 20% by number of the hydroxyl functions of these guar gums bear trialkylammonium cationic groups.

Among these trialkylammonium groups, mention may most particularly be made of the trimethylammonium and triethylammonium groups.

Even more preferentially, these groups represent from 5% to 20% by weight relative to the total weight of the modified guar gum.

According to the invention, a guar gum modified with 2,3-epoxypropyltrimethylammonium chloride is preferably used.

These guar gums modified with cationic groups are products already known *per se* and are, for example, described in patents US 3 589 578 and US 4 013 307. Such products are moreover notably sold under the trade names Jaguar C13 S, Jaguar C 15 and Jaguar C 17 by the company Meyhall.

Preferably, the guar gums are chosen from nonionic guar gums, more preferentially nonionic guar gums modified with hydroxyalkyl groups, more preferentially still hydroxypropyl guar.

The total content of guar gums preferably ranges from 0.01% to 20% by weight, more preferentially from 0.05% to 10% by weight, better still from 0.1% to 5% by weight, even better still from 0.5% to 3% by weight, relative to the total weight of the composition.

### Fatty substance

The composition according to the invention comprises one or more fatty substances, the total content of fatty substance being greater than or equal to 30% by weight, relative to the total weight of the composition.

Useful fatty substances according to the invention may be liquid fatty substances (or oils) and/or solid fatty substances. A liquid fatty substance is understood to be a fatty substance having a melting point of less than or equal to 25°C at atmospheric pressure (1.013×10⁵ Pa) and a solid fatty substance is understood to be a fatty substance having a melting point of greater than 25°C at atmospheric pressure (1.013×10⁵ Pa).

For the purposes of the present invention, the melting point corresponds to the temperature of the most endothermic peak observed on thermal analysis (differential scanning calorimetry or DSC) as described in the standard ISO 11357-3; 1999. The melting point may be measured using a differential scanning calorimeter (DSC), for example the calorimeter sold under the name MDSC 2920 by the company TA Instruments. In the present patent application, all the melting points are determined at atmospheric pressure (1.013×10⁵ Pa).

The term *"fatty substance"* is understood to mean an organic compound that is insoluble in water at 25°C and at atmospheric pressure (1.013×10⁵ Pa) (solubility of less than 5% by weight, and preferably less than 1% by weight, even more preferentially less than 0.1% by weight). They bear in their structure at least one hydrocarbon-based chain including at least 6 carbon atoms and/or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

Advantageously, the fatty substances that may be used in the present invention are neither (poly)oxyalkylenated nor (poly)glycerolated.

Preferably, useful fatty substances according to the invention are non-silicone.

The term "non-silicone fatty substance" is intended to mean a fatty substance not containing any Si-O bonds and the term "silicone fatty substance" is intended to mean a fatty substance containing at least one Si-O bond.

More particularly, the liquid fatty substance(s) according to the invention is/are chosen from C6 to C16 liquid hydrocarbons, liquid hydrocarbons comprising more than 16 carbon atoms, non-silicone oils of animal origin, oils of triglyceride type of plant or synthetic origin, fluoro oils, liquid fatty alcohols, liquid esters of fatty acid and/or of fatty alcohol other than triglycerides, and silicone oils, and mixtures thereof.

It is recalled that the fatty alcohols, esters and acids more particularly contain at least one saturated or unsaturated, linear or branched hydrocarbon-based group, comprising from 6 to 40 and better still from 8 to 30 carbon atoms, which is optionally substituted, in particular, with one or more hydroxyl groups (in particular 1 to 4). If they are unsaturated, these compounds may comprise one to three conjugated or unconjugated carbon-carbon double bonds.

As regards the C6 to C16 liquid hydrocarbons, these may be linear, branched, or optionally cyclic, and are preferably chosen from alkanes. Examples that may be mentioned include hexane, cyclohexane, undecane, dodecane, isododecane, tridecane or isoparaffins, such as isohexadecane or isodecane, and mixtures thereof.

The liquid hydrocarbons comprising more than 16 carbon atoms may be linear or branched, and of mineral or synthetic origin, and are preferably chosen from liquid paraffins or liquid petroleum jelly (or mineral oil), polydecenes, hydrogenated polyisobutene such as Parleam^{®}, and mixtures thereof.

A hydrocarbon-based oil of animal origin that may be mentioned is perhydrosqualene.

The triglyceride oils of plant or synthetic origin are preferably chosen from liquid triglycerides of fatty acids comprising from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarinerie Dubois or those sold under the names Miglyol^{®} 810, 812 and 818 by the company Dynamit Nobel, jojoba oil and shea butter oil, and mixtures thereof.

As regards the fluoro oils, they may be chosen from perfluoromethylcyclopentane and perfluoro-1,3-dimethylcyclohexane, sold under the names Flutec^{®} PC1 and Flutec^{®} PC3 by the company BNFL Fluorochemicals; perfluoro-1,2-dimethylcyclobutane; perfluoroalkanes such as dodecafluoropentane and tetradecafluorohexane, sold under the names PF 5050^{®} and PF 5060^{®} by the company 3M, or bromoperfluorooctyl sold under the name Foralkyl^{®} by the company Atochem; nonafluoromethoxybutane and nonafluoroethoxyisobutane; perfluoromorpholine derivatives such as 4-trifluoromethylperfluoromorpholine sold under the name PF 5052^{®} by the company 3M.

The liquid fatty alcohols that are suitable for use in the invention are more particularly chosen from linear or branched, saturated or unsaturated alcohols, preferably unsaturated or branched alcohols, comprising from 6 to 40 carbon atoms, preferably from 8 to 30 carbon atoms. Examples that may be mentioned include octyldodecanol, 2-butyloctanol, 2-hexyldecanol, 2-undecylpentadecanol, isostearyl alcohol, oleyl alcohol, linolenyl alcohol, ricinoleyl alcohol, undecylenyl alcohol and linoleyl alcohol, and mixtures thereof.

As regards the liquid esters of fatty acids and/or of fatty alcohols, other than the triglycerides mentioned previously, mention may be made especially of esters of saturated or unsaturated, linear C1 to C26 or branched C3 to C26 aliphatic mono- or polyacids and of saturated or unsaturated, linear C1 to C26 or branched C3 to C26 aliphatic mono- or polyalcohols, the total carbon number of the esters being greater than or equal to 6 and more advantageously greater than or equal to 10.

Preferably, for the esters of monoalcohols, at least one from among the alcohol and the acid from which the esters of the invention are derived is branched.

Among the monoesters, mention may be made of dihydroabietyl behenate; octyldodecyl behenate; isocetyl behenate; isostearyl lactate; lauryl lactate; linoleyl lactate; oleyl lactate; isostearyl octanoate; isocetyl octanoate; octyl octanoate; decyl oleate; isocetyl isostearate; isocetyl laurate; isocetyl stearate; isodecyl octanoate; isodecyl oleate; isononyl isononanoate; isostearyl palmitate; methyl acetyl ricinoleate; octyl isononanoate; 2-ethylhexyl isononanoate; octyldodecyl erucate; oleyl erucate; ethyl palmitate, isopropyl palmitate, such as 2-ethylhexyl palmitate, 2-octyldecyl palmitate; alkyl myristates such as isopropyl 2-octyldodecyl myristate, isobutyl stearate; 2-hexyldecyl laurate, and mixtures thereof.

Preferably, among the monoesters of monoacids and of monoalcohols, use will be made of ethyl palmitate and isopropyl palmitate, alkyl myristates such as isopropyl myristate or ethyl myristate, isocetyl stearate, 2-ethylhexyl isononanoate, isodecyl neopentanoate and isostearyl neopentanoate, and mixtures thereof.

Still within the context of this variant, esters of C4 to C22 dicarboxylic or tricarboxylic acids and of C1 to C22 alcohols and esters of monocarboxylic, dicarboxylic or tricarboxylic acids and of C2 to C26 dihydroxy, trihydroxy, tetrahydroxy or pentahydroxy alcohols may also be used.

Mention may notably be made of: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates, and mixtures thereof.

The composition may also comprise, as fatty ester, sugar esters and diesters of C6 to C30 and preferably C12 to C22 fatty acids. It is recalled that the term "sugar" refers to oxygen-bearing hydrocarbon-based compounds bearing several alcohol functions, with or without aldehyde or ketone functions, and which include at least 4 carbon atoms. These sugars may be monosaccharides, oligosaccharides or polysaccharides.

Examples of suitable sugars that may be mentioned include sucrose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C6 to C30 and preferably C12 to C22 fatty acids. If they are unsaturated, these compounds may comprise one to three conjugated or unconjugated carbon-carbon double bonds.

The esters according to this variant may also be chosen from mono-, di-, tri- and tetraesters, polyesters, and mixtures thereof.

These esters may be, for example, oleates, laurates, palmitates, myristates, behenates, cocoates, stearates, linoleates, linolenates, caprates, arachidonates or mixtures thereof, for instance especially the mixed oleopalmitate, oleostearate and palmitostearate esters.

More particularly, use is made of monoesters and diesters and especially sucrose, glucose or methylglucose mono- or dioleates, -stearates, -behenates, -oleopalmitates, -linoleates, - linolenates and -oleostearates, and mixtures thereof.

An example that may be mentioned is the product sold under the name Glucate^{®} DO by the company Amerchol, which is a methylglucose dioleate.

Preferably, use will be made of a liquid ester of a monoacid and of a monoalcohol.

The silicone oils that may be used in the composition A according to the present invention may be volatile or nonvolatile, cyclic, linear or branched silicone oils, which are unmodified or modified with organic groups, and preferably have a viscosity from 5×10⁻⁶ to 2.5 m²/s at 25°C, and preferably 1×10⁻⁵ to 1 m²/s.

Preferably, the silicone oils are chosen from polydialkylsiloxanes, especially polydimethylsiloxanes (PDMS), and liquid polyorganosiloxanes including at least one aryl group.

These silicone oils may also be organomodified. The organomodified silicone oils that may be used in accordance with the invention are preferably liquid silicones as defined previously and including in their structure one or more organofunctional groups attached via a hydrocarbon-based group, chosen, for example, from amine groups and alkoxy groups.

Organopolysiloxanes are defined in greater detail in Walter Noll's "Chemistry and Technology of Silicones" (1968), Academic Press. They may be volatile or non-volatile.

When they are volatile, the silicone oils are more particularly chosen from those with a boiling point of between 60°C and 260°C, and even more particularly from:
(i) cyclic polydialkylsiloxanes including from 3 to 7 and preferably from 4 to 5 silicon atoms. These are, for example, octamethylcyclotetrasiloxane sold notably under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, and Silbione^{®} 70045 V5 by Rhodia, and mixtures thereof.

Mention may also be made of cyclocopolymers of the dimethylsiloxane/methylalkylsiloxane type, such as Volatile Silicone^{®} FZ 3109 sold by the company Union Carbide.

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by Toray Silicone. Silicones falling within this category are also described in the article published in Cosmetics and Toiletries, Vol. 91, Jan. 76, pp. 27-32, Todd & Byers, "Volatile Silicone Fluids for Cosmetics".

Non-volatile polydialkylsiloxanes are preferably used.

These silicone oils are more particularly chosen from polydialkylsiloxanes, among which mention may be made mainly of polydimethylsiloxanes bearing trimethylsilyl end groups. The viscosity of the silicones is measured at 25°C according to the standard ASTM 445, Appendix C.

Among these polydialkylsiloxanes, mention may be made, in a nonlimiting manner, of the following commercial products:
- the Silbione^{®} oils of the 47 and 70 047 series or the Mirasil^{®} oils sold by Rhodia, such as, for example, the oil 70 047 V 500 000;
- the oils of the Mirasil^{®} series sold by the company Rhodia;
- the oils of the 200 series from Dow Corning, such as DC200 having a viscosity of 60 000 mm²/s;
- the Viscasil^{®} oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

The organomodified silicones that may be used in accordance with the invention are silicones as defined previously and comprising in their structure one or more organofunctional groups attached via a hydrocarbon-based group.

As regards the liquid polyorganosiloxanes including at least one aryl group, they may especially be polydiphenylsiloxanes, and polyalkylarylsiloxanes functionalized with the organofunctional groups mentioned previously.

The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.

Among these polyalkylarylsiloxanes, examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Among the organomodified silicones, mention may be made of polyorganosiloxanes including:
- substituted or unsubstituted amine groups, such as the products sold under the names GP 4 Silicone Fluid and GP 7100 by the company Genesee or the products sold under the names Q2 8220 and Dow Corning 929 or 939 by the company Dow Corning. The substituted amine groups are in particular C1 to C4 aminoalkyl groups;
- alkoxy groups,
- hydroxyl groups.

The solid fatty substances according to the invention preferably have a viscosity of greater than 2 Pa.s, measured at 25°C and at a shear rate of 1 s⁻¹.

The solid fatty substance(s) is/are preferably chosen from solid fatty acids, solid fatty alcohols, solid esters of fatty acids and/or of fatty alcohols, waxes, ceramides and mixtures thereof.

The term "fatty acids" is intended to mean a long-chain carboxylic acid comprising from 6 to 40 carbon atoms, preferably from 8 to 30 carbon atoms. The solid fatty acids according to the invention preferentially comprise from 10 to 30 carbon atoms and better still from 14 to 22 carbon atoms. They may optionally be hydroxylated. These fatty acids are neither oxyalkylenated nor glycerolated.

The solid fatty acids that may be used in the present invention are especially chosen from myristic acid, cetylic acid, stearylic acid, palmitic acid, arachidic acid, stearic acid, lauric acid, behenic acid, 12-hydroxystearic acid, and mixtures thereof.

Particularly preferably, the fatty acid(s) is/are chosen from behenic acid and arachidic acid.

The term "fatty alcohol" is intended to mean a long-chain aliphatic alcohol comprising from 6 to 40 carbon atoms, preferably from 8 to 30 carbon atoms, and comprising at least one hydroxyl group OH. These fatty alcohols are neither oxyalkylenated nor glycerolated.

The solid fatty alcohols may be saturated or unsaturated, and linear or branched, and include from 8 to 40 carbon atoms, preferably from 10 to 30 carbon atoms. Preferably, the solid fatty alcohols have the structure R-OH with R denoting a linear alkyl group, optionally substituted with one or more hydroxyl groups, comprising from 8 to 40, preferentially from 10 to 30 carbon atoms, better still from 10 to 30, or even from 12 to 24 and even better still from 14 to 22 carbon atoms.

The solid fatty alcohols that may be used are preferably chosen from saturated or unsaturated, linear or branched, preferably linear and saturated, (mono)alcohols including from 8 to 40 carbon atoms, better still from 10 to 30, or even from 12 to 24 and even better still from 14 to 22 carbon atoms.

The solid fatty alcohols that may be used may be chosen, alone or as a mixture, from: myristyl alcohol (or 1-tetradecanol); cetyl alcohol (or 1-hexadecanol); stearyl alcohol (or 1-octadecanol); arachidyl alcohol (or 1-eicosanol); behenyl alcohol (or 1-docosanol); lignoceryl alcohol (or 1-tetracosanol); ceryl alcohol (or 1-hexacosanol); montanyl alcohol (or 1-octacosanol); myricyl alcohol (or 1-triacontanol).

Preferentially, the solid fatty alcohol is chosen from cetyl alcohol, stearyl alcohol, behenyl alcohol, myristyl alcohol, arachidyl alcohol, and mixtures thereof, such as cetylstearyl alcohol or cetearyl alcohol. Particularly preferably, the solid fatty alcohol is behenyl alcohol.

The solid esters of a fatty acid and/or of a fatty alcohol that may be used are preferably chosen from esters derived from a C9-C26 carboxylic fatty acid and/or from a C9-C26 fatty alcohol.

Preferably, these solid fatty esters are esters of a linear or branched, saturated carboxylic acid including at least 10 carbon atoms, preferably from 10 to 30 carbon atoms and more particularly from 12 to 24 carbon atoms, and of a linear or branched, saturated monoalcohol, including at least 10 carbon atoms, preferably from 10 to 30 carbon atoms and more particularly from 12 to 24 carbon atoms. The saturated carboxylic acids may be optionally hydroxylated, and are preferably monocarboxylic acids.

Esters of C4-C22 dicarboxylic or tricarboxylic acids and of C1-C22 alcohols and esters of monocarboxylic, dicarboxylic or tricarboxylic acids and of C2-C26 dihydroxylated, trihydroxylated, tetrahydroxylated or pentahydroxylated alcohols may also be used.

Mention may in particular be made of octyldodecyl behenate, isocetyl behenate, cetyl lactate, stearyl octanoate, octyl octanoate, cetyl octanoate, decyl oleate, hexyl stearate, octyl stearate, myristyl stearate, cetyl stearate, stearyl stearate, octyl pelargonate, cetyl myristate, myristyl myristate, stearyl myristate, diethyl sebacate, diisopropyl sebacate, diisopropyl adipate, di-n-propyl adipate, dioctyl adipate, dioctyl maleate, octyl palmitate, myristyl palmitate, cetyl palmitate, stearyl palmitate, and mixtures thereof.

Preferably, the solid esters of a fatty acid and/or of a fatty alcohol are chosen from C9-C26 alkyl palmitates, notably myristyl palmitate, cetyl palmitate or stearyl palmitate; C9-C26 alkyl myristates, such as cetyl myristate, stearyl myristate and myristyl myristate; and C9-C26 alkyl stearates, notably myristyl stearate, cetyl stearate and stearyl stearate; and mixtures thereof.

For the purposes of the present invention, a wax is a lipophilic compound, which is solid at 25°C and atmospheric pressure, with a reversible solid/liquid change of state, having a melting point greater than about 40°C, which may range up to 200°C, and having in the solid state an anisotropic crystal organization. In general, the size of the wax crystals is such that the crystals diffract and/or scatter light, giving the composition that comprises them a more or less opaque cloudy appearance. By bringing the wax to its melting point, it is possible to make it miscible with oils and to form a microscopically homogeneous mixture, but on returning the temperature of the mixture to ambient temperature, recrystallization of the wax, which is microscopically and macroscopically detectable (opalescence), is obtained.

In particular, the waxes that are suitable for use in the invention may be chosen from waxes of animal, plant or mineral origin, non-silicone synthetic waxes, and mixtures thereof.

Mention may be made notably of hydrocarbon-based waxes, for instance beeswax, notably of biological origin, lanolin wax and Chinese insect waxes; rice bran wax, carnauba wax, candelilla wax, ouricury wax, esparto grass wax, berry wax, shellac wax, Japan wax and sumac wax; montan wax, orange wax and lemon wax, microcrystalline waxes, paraffins and ozokerite; polyethylene waxes, the waxes obtained by Fischer-Tropsch synthesis and waxy copolymers, and also esters thereof.

Mention may additionally be made of C20 to C60 microcrystalline waxes, such as Microwax HW.

Mention may also be made of the MW 500 polyethylene wax sold under the reference Permalen 50-L polyethylene.

Mention may also be made of the waxes obtained by catalytic hydrogenation of animal or plant oils containing linear or branched C8-C32 fatty chains. Among these waxes mention may especially be made of isomerized jojoba oil such as the trans-isomerized partially hydrogenated jojoba oil, especially the product manufactured or sold by the company Desert Whale under the commercial reference Iso-Jojoba-50^{®}, hydrogenated sunflower oil, hydrogenated castor oil, hydrogenated coconut oil, hydrogenated lanolin oil and bis(1,1,1-trimethylolpropane) tetrastearate, especially the product sold under the name Hest 2T-4S^{®} by the company Heterene.

The waxes obtained by hydrogenation of castor oil esterified with cetyl alcohol, such as those sold under the names Phytowax Castor 16L64^{®} and 22L73^{®} by the company Sophim, may also be used.

A wax that may also be used is a C20-C40 alkyl (hydroxystearyloxy)stearate (the alkyl group containing from 20 to 40 carbon atoms), alone or as a mixture. Such a wax is especially sold under the names Kester Wax K 82 P^{®}, Hydroxypolyester K 82 P^{®} and Kester Wax K 80 P^{®} by the company Koster Keunen.

It is also possible to use microwaxes in the compositions of the invention; mention may be made especially of carnauba microwaxes, such as the product sold under the name MicroCare 350^{®} by the company Micro Powders, synthetic-wax microwaxes, such as the product sold under the name MicroEase 114S^{®} by the company Micro Powders, microwaxes constituted of a mixture of carnauba wax and polyethylene wax, such as the products sold under the names Micro Care 300^{®} and 310^{®} by the company Micro Powders, microwaxes constituted of a mixture of carnauba wax and of synthetic wax, such as the product sold under the name Micro Care 325^{®} by the company Micro Powders, polyethylene microwaxes, such as the products sold under the names Micropoly 200^{®}, 220^{®}, 220L^{®} and 250S^{®} by the company Micro Powders, and polytetrafluoroethylene microwaxes, such as the products sold under the names Microslip 519^{®} and 519 L^{®} by the company Micro Powders.

The waxes are preferably chosen from mineral waxes, for instance paraffin, petroleum jelly, lignite or ozokerite wax; plant waxes, for instance cocoa butter or cork fibre or sugar cane waxes, olive tree wax, rice wax, hydrogenated jojoba wax, ouricury wax, carnauba wax, candelilla wax, esparto grass wax, or absolute waxes of flowers, such as the essential wax of blackcurrant blossom sold by the company Bertin (France); waxes of animal origin, for instance beeswaxes or modified beeswaxes (cera bellina), spermaceti, lanolin wax and lanolin derivatives; microcrystalline waxes; and mixtures thereof.

The ceramides, or ceramide analogues such as glycoceramides, which may be used in the compositions according to the invention, are known; mention may in particular be made of ceramides of classes I, II, III and V according to the Dawning classification.

The ceramides or analogues thereof that may be used preferably correspond to the following formula: R³CH(OH)CH(CH₂OR²)(NHCOR¹), in which:
**R¹** denotes a linear or branched, saturated or unsaturated alkyl group, derived from C₁₄-C₃₀ fatty acids, it being possible for this group to be substituted with a hydroxyl group in the alpha position, or a hydroxyl group in the omega position esterified with a saturated or unsaturated C₁₆-C₃₀ fatty acid;
**R²** denotes a hydrogen atom, a (glycosyl)n group, a (galactosyl)m group or a sulfogalactosyl group, in which n is an integer ranging from 1 to 4 and m is an integer ranging from 1 to 8;
**R³** denotes a C₁₅-C₂₆ hydrocarbon-based group, saturated or unsaturated in the alpha position, it being possible for this group to be substituted with one or more C₁-C₁₄ alkyl groups; it being understood that in the case of natural ceramides or glycoceramides, R³ may also denote a C₁₅-C₂₆ alpha-hydroxyalkyl group, the hydroxyl group being optionally esterified by a C₁₆-C₃₀ alpha-hydroxy acid.

The ceramides that are more particularly preferred are the compounds for which **R¹** denotes a saturated or unsaturated alkyl derived from C₁₆-C₂₂ fatty acids; **R²** denotes a hydrogen atom and R³ denotes a saturated linear C₁₅ group.

Preferentially, use is made of ceramides for which **R¹** denotes a saturated or unsaturated alkyl group derived from C₁₄-C₃₀ fatty acids; **R²** denotes a galactosyl or sulfogalactosyl group; and **R³** denotes a -CH=CH-(CH2)₁₂-CH3 group.

Use may also be made of the compounds for which **R¹** denotes a saturated or unsaturated alkyl radical derived from C₁₂-C₂₂ fatty acids; **R²** denotes a galactosyl or sulfogalactosyl radical and **R³** denotes a saturated or unsaturated C₁₂-C₂₂ hydrocarbon-based radical and preferably a -CH=CH-(CH2)₁₂-CH3 group.

As compounds that are particularly preferred, mention may also be made of 2-N-linoleoylaminooctadecane-1,3-diol; 2-N-oleoylaminooctadecane-1,3-diol; 2-N-palmitoylaminooctadecane-1,3-diol; 2-N-stearoylaminooctadecane-1,3-diol; 2-N-behenoylaminooctadecane-1,3-diol; 2-N-[2-hydroxypalmitoyl]aminooctadecane-1,3-diol; 2-N-stearoylaminooctadecane-1,3,4-triol and in particular N-stearoylphytosphingosine, 2-N-palmitoylaminohexadecane-1,3-diol, N-linoleoyldihydrosphingosine, N-oleoyldihydrosphingosine, N-palmitoyldihydrosphingosine, N-stearoyldihydrosphingosine, and N-behenoyldihydrosphingosine, N-docosanoyl-N-methyl-D-glucamine, cetylic acid N-(2-hydroxyethyl)-N-(3-cetyloxy-2-hydroxypropyl)amide and bis(N-hydroxyethyl-N-cetyl)malonamide; and mixtures thereof. N-Oleoyldihydrosphingosine will preferably be used.

The solid fatty substances are preferably chosen from solid fatty acids, solid fatty alcohols and mixtures thereof.

According to a preferred embodiment, useful fatty substances according to the invention are chosen from liquid fatty substances, more preferentially from liquid hydrocarbons containing more than 16 carbon atoms, plant oils, liquid fatty alcohols and liquid fatty esters, silicone oils and mixtures thereof.

Preferentially, the liquid fatty substance(s) is/are chosen from liquid hydrocarbons comprising more than 16 carbon atoms, in particular liquid petroleum jelly.

Preferably, the total content of the fatty substance(s) is greater than or equal to 35% by weight, more preferentially greater than or equal to 40% by weight, better still greater than or equal to 45% by weight, even better still greater than or equal to 50% by weight, relative to the total weight of the composition.

Preferably, the total content of the liquid fatty substance(s) is greater than or equal to 30% by weight, preferably greater than or equal to 35% by weight, more preferentially greater than or equal to 40% by weight, better still greater than or equal to 45% by weight, even better still greater than or equal to 50% by weight, relative to the total weight of the composition.

In a particularly preferred embodiment, the total content of the liquid fatty substance(s) chosen from liquid hydrocarbons comprising more than 16 carbon atoms is greater than or equal to 30% by weight, preferably greater than or equal to 35% by weight, more preferentially greater than or equal to 40% by weight, better still greater than or equal to 45% by weight, even better still greater than or equal to 50% by weight, relative to the total weight of the composition.

### Couplers

The composition according to the invention may comprise at least one oxidation coupler (or coupling agent) advantageously chosen from those conventionally used in the dyeing of keratin fibres.

Preferably, the composition according to the invention comprises at least one oxidation coupler.

Among oxidation couplers, mention may be made in particular of meta-phenylenediamines, meta-aminophenols, meta-diphenols, naphthalene-based coupling agents and heterocyclic coupling agents, and also the corresponding addition salts.

Mention may be made, for example, of 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, hydroxyethyl-3,4-methylenedioxyaniline, 2-amino-5-ethylphenol, 2,6-bis(β-hydroxyethylamino)toluene, 6-hydroxyindoline, 2,6-dihydroxy-4-methylpyridine, 1-H-3-methylpyrazol-5-one, 1-phenyl-3-methylpyrazol-5-one, 2,6-dimethylpyrazolo[1,5-b][1,2,4]triazole, 2,6-dimethyl[3,2-c][1,2,4]triazole and 6-methylpyrazolo[1,5-a]benzimidazole, 2-methyl-5-aminophenol, 5-N-(β-hydroxyethyl)amino-2-methylphenol, 3-aminophenol and 3-amino-2-chloro-6-methylphenol, the corresponding addition salts with an acid and the corresponding mixtures.

In general, the addition salts of the couplers that may be used in the context of the invention are chosen in particular from the addition salts with an acid such as the hydrochlorides, hydrobromides, sulfates, citrates, succinates, tartrates, lactates, tosylates, benzenesulfonates, phosphates and acetates.

According to a preferred embodiment, the composition according to the present invention comprises one or more couplers chosen from: 6-hydroxybenzomorpholine of formula (II), its addition salts, its solvates and/or the solvates of its salts, hydroxyethyl-3,4-methylenedioxyaniline of formula (III), its addition salts, its solvates and/or the solvates of its salts, 2-amino-5-ethylphenol of formula (IV), its addition salts, its solvates and/or the solvates of its salts, and mixtures thereof.

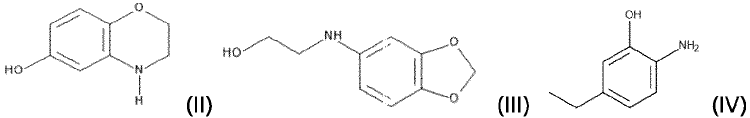

The addition salts of the compounds of formulae (II), (III) and (IV) are chosen especially from the addition salts with an acid, such as the hydrochlorides, hydrobromides, sulfates, citrates, succinates, tartrates, lactates, tosylates, benzenesulfonates, phosphates and acetates, and the addition salts with a base such as sodium hydroxide, potassium hydroxide, aqueous ammonia, amines or alkanolamines.

In a particular embodiment, the composition according to the invention is free from oxidation couplers chosen from resorcinol, 2-methylresorcinol, 4-chlororesorcinol, their addition salts, their solvates and the solvates of their salts.

Moreover, the solvates of the compounds of formulae (II), (III) and (IV) more particularly represent the hydrates of these compounds and/or the combination of these compounds with a linear or branched C1 to C4 alcohol such as methanol, ethanol, isopropanol or n-propanol. Preferably, the solvates are hydrates.

When the composition comprises one or more oxidation couplers, the total content of the coupler(s) preferably ranges from 0.001% to 20% by weight, more preferentially from 0.005% to 15% by weight, better still from 0.01% to 10% by weight, even better still from 0.05% to 5% by weight, better yet from 0.1% to 3% by weight, relative to the total weight of the composition.

When the composition comprises one or more oxidation couplers chosen from the couplers of formulae (II), (III) and (IV) and their addition salts, their solvates and/or the solvates of their salts, the total content thereof preferably ranges from 0.001 % to 20% by weight, more preferentially from 0.005% to 15% by weight, better still from 0.01% to 10% by weight, even better still from 0.05% to 5% by weight, better yet from 0.1% to 3% by weight, relative to the total weight of the composition.

### Surfactants

The composition according to the present invention may comprise one or more surfactants. These may be chosen from anionic surfactants, nonionic surfactants and cationic surfactants and/or mixtures thereof.

Preferably, the composition according to the present invention comprises one or more surfactants.

The term **"anionic surfactant"** is understood to mean a surfactant including, as ionic or ionizable groups, only anionic groups. These anionic groups are preferably chosen from the following groups: CO₂H, CO₂-, SO₃H, SO₃⁻, OSO₃H, OSO₃⁻, H₂PO₃, HPO₃⁻, PO₃²⁻, H₂PO₂, HPO₂⁻, PO₂²⁻, POH and PO-.

As examples of anionic surfactants that can be used in the composition according to the invention, mention may be made of alkyl sulfates, alkyl ether sulfates, alkylamido ether sulfates, alkylaryl polyether sulfates, monoglyceride sulfates, alkylsulfonates, alkylamidesulfonates, alkylarylsulfonates, α-olefinsulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-(C1-C4)alkyl N-acyltaurates, salts of alkyl monoesters of polyglycoside-polycarboxylic acids, acyl lactylates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds (unless specified otherwise) generally comprising from 6 to 24 carbon atoms and the aryl group generally denoting a phenyl group.

These compounds may be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside-citrates, C₆-C₂₄ alkyl polyglycoside-tartrates and C₆-C₂₄ alkyl polyglycoside-sulfosuccinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salt.

Examples of amino alcohol salts that may notably be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

The anionic surfactants optionally present may be mild anionic surfactants, i.e. anionic surfactants without a sulfate function.

As regards mild anionic surfactants, mention may be made in particular of the following compounds and salts thereof, and also mixtures thereof: polyoxyalkylenated alkyl ether carboxylic acids, polyoxyalkylenated alkylaryl ether carboxylic acids, polyoxyalkylenated alkylamido ether carboxylic acids, in particular those comprising 2 to 50 ethylene oxide groups, alkyl D-galactoside uronic acids, acyl sarcosinates, acyl glutamates and alkylpolyglycoside carboxylic esters.

Use may be made most particularly of polyoxyalkylenated alkyl ether carboxylic acids, for instance lauryl ether carboxylic acid (4.5 EO), sold, for example, under the name Akypo RLM 45 CA from Kao.

Among the anionic surfactants mentioned above, use is preferably made of sulfated surfactants such as alkyl sulfates or alkyl ether sulfates, and acyl glutamates, more preferentially sulfated anionic surfactants, even more preferentially alkyl sulfates.

The nonionic surfactant(s) that may be used in the composition of the present invention are in particular described, for example, in the Handbook of Surfactants by M.R. Porter, published by Blackie & Son (Glasgow and London), 1991, pages 116-178.

Examples of nonionic surfactants that may be mentioned include the following compounds, alone or as a mixture:
- oxyalkylenated (C₈-C₂₄)alkylphenols;
- saturated or unsaturated, linear or branched, oxyalkylenated or glycerolated C₈-C₄₀ alcohols, preferably comprising one or two fatty chains;
- saturated or unsaturated, linear or branched, oxyalkylenated C₈ to C₃₀ fatty acid amides;
- esters of saturated or unsaturated, linear or branched, C₈ to C₃₀ acids and of polyethylene glycols;
- esters of saturated or unsaturated, linear or branched, C₈ to C₃₀ acids and of sorbitol which are preferably oxyethylenated;
- esters of fatty acids and of sucrose;
- C₈-C₃₀ fatty acid esters of sorbitan,
- (C₈-C₃₀)alkyl(poly)glucosides, (C₈-C₃₀)alkenyl(poly)glucosides, which are optionally oxyalkylenated (0 to 10 oxyalkylene units) and comprising from 1 to 15 glucose units, (C₈-C₃₀)alkyl(poly)glucoside esters;
- saturated or unsaturated oxyethylenated plant oils;
- condensates of ethylene oxide and/or of propylene oxide;
- *N*-(C₈-C₃₀)alkylglucamine and *N*-(C₈-C₃₀)acylmethylglucamine derivatives;
- amine oxides.

They are notably chosen from alcohols, α-diols and (C₁-C₂₀)alkylphenols, these compounds being ethoxylated, propoxylated or glycerolated and bearing at least one fatty chain comprising, for example, from 8 to 24 carbon atoms, preferably from 8 to 18 carbon atoms, the number of ethylene oxide or propylene oxide groups possibly ranging notably from 1 to 200, and the number of glycerol groups possibly ranging notably from 1 to 30.

Mention may also be made of condensates of ethylene oxide and of propylene oxide with fatty alcohols, ethoxylated fatty amides preferably containing from 1 to 30 ethylene oxide units, polyglycerolated fatty amides comprising on average from 1 to 5, and in particular from 1.5 to 4, glycerol groups, ethoxylated fatty acid esters of sorbitan containing from 1 to 30 ethylene oxide units, fatty acid esters of sucrose, fatty acid esters of polyethylene glycol, (C₆-C₂₄ alkyl)polyglycosides, oxyethylenated plant oils, N-(C₆-C₂₄ alkyl)glucamine derivatives, amine oxides such as (C₁₀-C₁₄ alkyl)amine oxides or N-(C₁₀-C₁₄ acyl)aminopropylmorpholine oxides.

The C₈-C₃₀, preferably C₁₂-C₂₂, fatty acid esters (especially monoesters, diesters and triesters) of sorbitan may be chosen from:
sorbitan caprylate; sorbitan cocoate; sorbitan isostearate; sorbitan laurate; sorbitan oleate; sorbitan palmitate; sorbitan stearate; sorbitan diisostearate; sorbitan dioleate; sorbitan distearate; sorbitan sesquicaprylate; sorbitan sesquiisostearate; sorbitan sesquioleate; sorbitan sesquistearate; sorbitan triisostearate; sorbitan trioleate; sorbitan tristearate.

The polyoxyethylenated C₈-C₃₀ (preferably C₁₂-C₁₈) fatty acid esters (especially monoesters, diesters and triesters) of sorbitan especially containing from 2 to 20 mol of ethylene oxide may be chosen from polyoxyethylenated esters of C₁₂-C₁₈ fatty acids, in particular lauric, myristic, cetylic or stearic acid, and of sorbitan especially containing from 2 to 30 mol of ethylene oxide, such as:
polyoxyethylenated sorbitan monolaurate (4 EO) (POLYSORBATE-21),
polyoxyethylenated sorbitan monolaurate (20 EO) (POLYSORBATE-20),
polyoxyethylenated sorbitan monopalmitate (20 EO) (POLYSORBATE-40),
polyoxyethylenated sorbitan monostearate (20 EO) (POLYSORBATE-60),
polyoxyethylenated sorbitan monostearate (4 EO) (POLYSORBATE-61),
polyoxyethylenated sorbitan monooleate (20 EO) (POLYSORBATE-80),
polyoxyethylenated sorbitan monooleate (5 EO) (POLYSORBATE-81),
polyoxyethylenated sorbitan tristearate (20 EO) (POLYSORBATE-65),
polyoxyethylenated sorbitan trioleate (20 EO) (POLYSORBATE-85).

The polyoxyethylenated C₈-C₃₀ (preferably C₁₂-C₁₈) fatty acid esters (especially monoesters, diesters, triesters and tetraesters) of sorbitan, especially containing from 2 to 20 mol of ethylene oxide, may be chosen from polyoxyethylenated esters, especially containing from 2 to 20 mol of ethylene oxide of C₁₂-C₁₈ fatty acids, in particular lauric, myristic, cetylic or stearic acid, and of sorbitan, such as:
- the ester polyoxyethylenated with 20 EO of sorbitan and of cocoic acid (PEG-20 Sorbitan Cocoate);
- the polyoxyethylenated esters (especially containing from 2 to 20 EO) of sorbitan and of isostearic acid (such as PEG-2 Sorbitan Isostearate; PEG-5 Sorbitan Isostearate; PEG-20 Sorbitan Isostearate such as the product sold under the name Nikkol TI 10 V by the company Nikkol),
- the polyoxyethylenated esters (especially containing from 2 to 20 EO) of sorbitan and of lauric acid (such as PEG-10 Sorbitan Laurate),
- the polyoxyethylenated esters (especially containing from 2 to 20 EO) of sorbitan and of oleic acid containing 10 oxyethylene groups (such as PEG-6 Sorbitan Oleate; PEG-20 sorbitan oleate),
- the polyoxyethylenated esters (especially containing from 3 to 20 EO) of sorbitan and of stearic acid (such as PEG-3 Sorbitan Stearate; PEG-4 Sorbitan Stearate; PEG-6 Sorbitan Stearate).

The nonionic surfactant(s) is/are preferably chosen from ethoxylated C₈-C₂₄ fatty alcohols comprising from 1 to 200 ethylene oxide groups, ethoxylated C₈-C₃₀ fatty acid esters of sorbitan having from 1 to 30 ethylene oxide units, (C₆-C₂₄ alkyl)polyglycosides, and mixtures thereof, better still from (C₆-C₂₄ alkyl)polyglycosides, even better still from (C₆-C₂₄ alkyl)polyglycosides such as: coco glucoside, caprylyl/capryl glucoside, lauryl glucoside and decyl glucoside.

The cationic surfactant(s) that may be used in the composition according to the invention are generally chosen from optionally polyoxyalkylenated primary, secondary or tertiary fatty amines, quaternary ammonium salts, and mixtures thereof.

The fatty amines generally comprise at least one C₈-C₃₀ hydrocarbon-based chain. Among the fatty amines that may be used according to the invention, examples that may be mentioned include stearylamidopropyldimethylamine and distearylamine.

Examples of quaternary ammonium salts that may notably be mentioned include:
- those corresponding to the general formula (X) below: 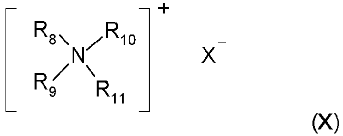 in which the groups R₈ to R₁₁, which may be identical or different, represent a linear or branched aliphatic group comprising from 1 to 30 carbon atoms, or an aromatic group such as aryl or alkylaryl, at least one of the groups R₈ to R₁₁ comprising from 8 to 30 carbon atoms and preferably from 12 to 24 carbon atoms. The aliphatic groups may include heteroatoms such as especially oxygen, nitrogen, sulfur and halogens.

The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X- is an anion chosen from the group of halides, phosphates, acetates, lactates, (C₁-C₄)alkyl sulfates and (C₁-C₄)alkylsulfonates or (C₁-C₄)alkylarylsulfonates.

Among the quaternary ammonium salts of formula (X), preference is given, firstly, to tetraalkylammonium chlorides, for instance dialkyldimethylammonium or alkyltrimethylammonium chlorides in which the alkyl group includes from about 12 to 22 carbon atoms, in particular behenyltrimethylammonium chloride, distearyldimethylammonium chloride, cetyltrimethylammonium chloride or benzyldimethylstearylammonium chloride, or, secondly, to distearoylethylhydroxyethylmethylammonium methosulfate, dipalmitoylethylhydroxyethylammonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl^{®} 70 by the company Van Dyk;
- quaternary ammonium salts of imidazoline, for instance those of formula (XI) below: 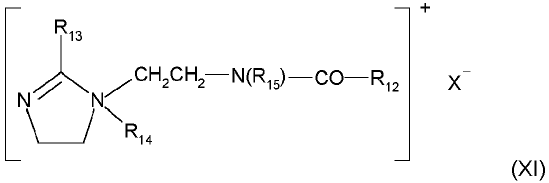 in which R12 represents an alkenyl or alkyl group including from 8 to 30 carbon atoms, for example tallow fatty acid derivatives, R13 represents a hydrogen atom, a C₁-C₄ alkyl group or an alkenyl or alkyl group including from 8 to 30 carbon atoms, R14 represents a C₁-C₄ alkyl group, R15 represents a hydrogen atom or a C₁-C₄ alkyl group, and X⁻ is an anion chosen from the group of halides, phosphates, acetates, lactates, (C₁-C₄)alkyl sulfates, and (C₁-C₄)alkylsulfonates or (C₁-C₄)alkylarylsulfonates.

Preferably, R12 and R13 denote a mixture of alkenyl or alkyl groups including from 12 to 21 carbon atoms, for example tallow fatty acid derivatives, R14 denotes a methyl group and R15 denotes a hydrogen atom. Such a product is sold, for example, under the name Rewoquat^{®} W 75 by the company Rewo;
- quaternary diammonium or triammonium salts, in particular of formula (XII) below: 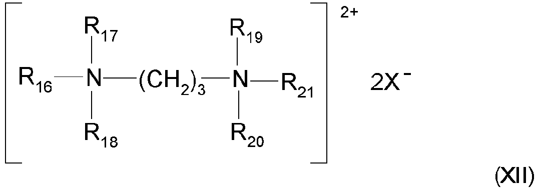 in which R16 denotes an alkyl group containing approximately from 16 to 30 carbon atoms, which is optionally hydroxylated and/or interrupted with one or more oxygen atoms; R17 is chosen from hydrogen, an alkyl group containing from 1 to 4 carbon atoms or a group -(CH2)3-N+(R16a)(R17a)(R18a), R16a, R17a, R18a, **R18, R19,** R20 and **R21,** which may be identical or different, are chosen from hydrogen or an alkyl group containing from 1 to 4 carbon atoms, and X- is an anion chosen from the group of halides, acetates, phosphates, nitrates, (C1-C4)alkyl sulfates, (C1-C4)alkylsulfonates or (C1-C4)alkylarylsulfonates, in particular methyl sulfate and ethyl sulfate.

Such compounds are, for example, Finquat CT-P, sold by the company Finetex (Quaternium 89), and Finquat CT, sold by the company Finetex (Quaternium 75);
- quaternary ammonium salts containing one or more ester functions, for instance those of formula (XIII) below: 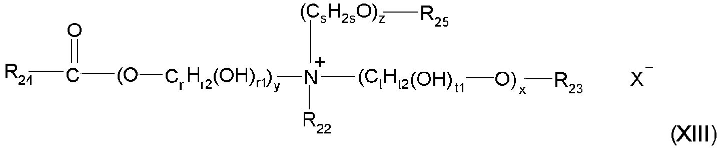 in which: R22 is chosen from C1-C6 alkyl groups and C1-C6 hydroxyalkyl or dihydroxyalkyl groups; R23 is chosen from: the group -C(O)R26, linear or branched, saturated or unsaturated C1-C22 hydrocarbon-based groups R27, or a hydrogen atom; R25 is chosen from: the group -C(O)R28, linear or branched, saturated or unsaturated C1-C6 hydrocarbon-based groups R29, or a hydrogen atom; R24, R26 and R28, which may be identical or different, are chosen from linear or branched, saturated or unsaturated C7-C21 hydrocarbon-based groups; r, s and t, which may be identical or different, are integers from 2 to 6; r1 and t1, which may be identical or different, are 0 or 1; r2 + r1 = 2 r and t1 + t2 = 2 t, y is an integer from 1 to 10, x and z, which may be identical or different, are integers from 0 to 10, X- is an organic or inorganic simple or complex anion, with the proviso that the sum x + y + z is from 1 to 15, that when x is 0 R23 denotes R27 and that when z is 0 R25 denotes R29.

The alkyl groups R22 may be linear or branched, and more particularly linear.

Preferably, R22 denotes a methyl, ethyl, hydroxyethyl or dihydroxypropyl group, and more particularly a methyl or ethyl group.

Advantageously, the sum x + y + z is from 1 to 10.

When R23 is a hydrocarbon-based group R27, it may be long and contain from 12 to 22 carbon atoms, or may be short and contain from 1 to 3 carbon atoms.

When R25 is a hydrocarbon-based group R29, it preferably contains 1 to 3 carbon atoms.

Advantageously, R24, R26 and R28, which may be identical or different, are chosen from linear or branched, saturated or unsaturated C11-C21 hydrocarbon-based groups, and more particularly from linear or branched, saturated or unsaturated C11-C21 alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X- is preferably a halide, preferably chloride, bromide or iodide, a (C1-C4)alkyl sulfate, (C1-C4)alkylsulfonate or (C1-C4)alkylarylsulfonate. However, use may be made of methanesulfonate, phosphate, nitrate, tosylate, an anion derived from an organic acid, such as acetate or lactate, or any other anion that is compatible with the ammonium bearing an ester function.

The anion X⁻ is even more particularly chloride, methyl sulfate or ethyl sulfate.

Use is made more particularly, in the composition according to the invention, of the ammonium salts of formula (XIII) in which: R22 denotes a methyl or ethyl group, x and y are equal to 1, z is equal to 0 or 1, r, s and t are equal to 2; R23 is chosen from: the group -C(O)R26, methyl, ethyl or C14-C22 hydrocarbon-based groups, or a hydrogen atom, R25 is chosen from: the group -C(O)R28, or a hydrogen atom, R24, R26 and R28, which may be identical or different, are chosen from linear or branched, saturated or unsaturated C13-C17 hydrocarbon-based groups, and preferably from linear or branched, saturated or unsaturated C13-C17 alkyl and alkenyl groups.

Advantageously, the hydrocarbon-based groups are linear.

Among the compounds of formula (XIII), examples that may be mentioned include salts, especially the chloride or methyl sulfate, of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are derived more particularly from a plant oil such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with mixtures of fatty acids of plant or animal origin, or by transesterification of the methyl esters thereof. This esterification is followed by quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin.

Such compounds are sold, for example, under the names Dehyquart^{®} by the company Henkel, Stepanquat^{®} by the company Stepan, Noxamium^{®} by the company CECA or Rewoquat^{®} WE 18 by the company Rewo-Witco.

The composition according to the invention may contain, for example, a mixture of quaternary ammonium monoester, diester and triester salts with a weight majority of diester salts.

Use may also be made of the ammonium salts containing at least one ester function that are described in patents US-A-4 874 554 and US-A-4 137 180.

Use may also be made of the behenoylhydroxypropyltrimethylammonium chloride sold, for example, by the company KAO under the name Quartamin BTC 131.

Preferably, the ammonium salts containing at least one ester function contain two ester functions.

Among the cationic surfactants, it is more particularly preferred to choose cetyltrimethylammonium, behenyltrimethylammonium and dipalmitoylethylhydroxyethyl-methylammonium salts, and mixtures thereof, and more particularly behenyltrimethylammonium chloride, cetyltrimethylammonium chloride, and dipalmitoylethylhydroxyethylammonium methosulfate, and mixtures thereof.

Preferably, the surfactant(s) is/are chosen from anionic surfactants, nonionic surfactants and mixtures thereof, preferentially from sulfated anionic surfactants, such as alkyl sulfates, ethoxylated C8-C24 fatty alcohols comprising from 1 to 200 ethylene oxide groups, ethoxylated C8-C30 fatty acid esters of sorbitan having from 1 to 30 ethylene oxide units, (C₆-C₂₄ alkyl)polyglycosides, and mixtures thereof.

More preferentially, the surfactant(s) is/are chosen from nonionic surfactants, better still from (C₆-C₂₄ alkyl)polyglycosides, even better still from (C₆-C₂₄ alkyl)polyglucosides such as: coco glucoside, caprylyl/capryl glucoside, lauryl glucoside and decyl glucoside.

When the composition comprises one or more surfactants, with preference, the total content of surfactant(s) in the composition preferably ranges from 0.01% to 15% by weight, more preferentially from 0.1% to 10% by weight, better still from 0.5% to 8% by weight, even better still from 1% to 6% by weight, relative to the total weight of the composition.

### Sequestrant

The composition according to the invention may comprise one or more sequestrants (or chelating agents).

Preferably, the composition according to the invention comprises at least one sequestrant.

The definition of a "sequestrant" (or "chelating agent") is well known to those skilled in the art and refers to a compound or a mixture of compounds capable of forming a chelate with a metal ion. A chelate is an inorganic complex in which a compound (the sequestrant or chelating agent) is coordinated to a metal ion, i.e. it forms one or more bonds with the metal ion (formation of a ring including the metal ion).

A sequestrant (or chelating agent) generally comprises at least two electron-donating atoms which enable the formation of bonds with the metal ion.

Within the context of the present invention, the sequestrant(s) may be chosen from carboxylic acids, preferably aminocarboxylic acids, phosphonic acids, preferably aminophosphonic acids, polyphosphoric acids, preferably linear polyphosphoric acids, salts thereof, and derivatives thereof.

The salts are in particular alkali metal, alkaline-earth metal, ammonium and substituted ammonium salts.

The following compounds may be mentioned as examples of sequestrants based on **carboxylic acids:** diethylenetriaminepentaacetic acid (DTPA), ethylenediaminedisuccinic acid (EDDS) and trisodium ethylenediaminedisuccinate such as Octaquest E30 from OCTEL, ethylenediaminetetraacetic acid (EDTA), and its salts such as disodium EDTA, tetrasodium EDTA, ethylenediamine-N,N'-diglutaric acid (EDDG), glycinamide-N,N'-disuccinic acid (GADS), 2-hydroxypropylenediamine-N,N'-disuccinic acid (HPDDS), ethylenediamine-N,N'-bis(ortho-hydroxyphenylacetic acid) (EDDHA), N,N'-bis(2-hydroxybenzyl)ethylenediamine-N,N'-diacetic acid (HBED), nitrilotriacetic acid (NTA), methylglycinediacetic acid (MGDA), N-2-hydroxyethyl-N,N-diacetic acid and glyceryliminodiacetic acid (as described in documents EP-A-317,542 and EP-A-399,133), iminodiacetic acid-N-2-hydroxypropylsulfonic acid and aspartic acid-N-carboxymethyl-N-2-hydroxypropyl-3-sulfonic acid (as described in EP-A-516,102), beta-alanine-N,N'-diacetic acid, aspartic acid-N,N'-diacetic acid, aspartic acid-N-monoacetic acid (described in EP-A-509,382), chelating agents based on iminodisuccinic acid (IDSA) (as described in EP-A-509,382), ethanoldiglycine acid, phosphonobutanetricarboxylic acid such as the compound sold by Bayer under the reference Bayhibit AM, and N,N-dicarboxymethylglutamic acid and salts thereof such as tetrasodium glutamate diacetate (GLDA) such as Dissolvine GL38 or 45S from Akzo Nobel.

The following compounds may be mentioned as examples of chelating agents based on mono- or polyphosphonic acid: diethylenetriaminepenta(methylenephosphonic acid) (DTPMP), ethane-1-hydroxy-1,1,2-triphosphonic acid (E1HTP), ethane-2-hydroxy-1,1,2-triphosphonic acid (E2HTP), ethane-1-hydroxy-1,1-diphosphonic acid (EHDP), ethane-1,1,2-triphosphonic acid (ETP), ethylenediaminetetramethylenephosphonic acid (EDTMP), hydroxyethane-1,1-diphosphonic acid (HEDP, or etidronic acid), and salts such as disodium etidronate, tetrasodium etidronate.

The following compounds may be mentioned as examples of chelating agents based on polyphosphoric acid: sodium tripolyphosphate (STP), tetrasodium diphosphate, hexametaphosphoric acid, sodium metaphosphate, phytic acid.

According to a particular embodiment, the sequestrant(s) useful according to the invention is/are phosphorus-based sequestrants, i.e. sequestrants which comprise one or more phosphorus atoms, preferably at least two phosphorus atoms.

The phosphorus-based sequestrants used in the composition according to the invention are preferably chosen from:
- inorganic phosphorus-based derivatives preferably chosen from alkali metal or alkaline-earth metal, preferably alkali metal, phosphates and pyrophosphates, such as sodium pyrophosphate, potassium pyrophosphate, sodium pyrophosphate decahydrate; and alkali metal or alkaline-earth metal, preferably alkali metal, polyphosphates, such as sodium hexametaphosphate, sodium polyphosphate, sodium tripolyphosphate, sodium trimetaphosphate; which are optionally hydrated, and mixtures thereof;
- organic phosphorus-based derivatives, such as organic (poly)phosphates and (poly)phosphonates, such as etidronic acid and/or alkali metal or alkaline-earth metal salts thereof, for instance tetrasodium etidronate, disodium etidronate, and mixtures thereof.

Preferably, the phosphorus-based sequestrant(s) is/are chosen from linear or cyclic compounds comprising at least two phosphorus atoms bonded together covalently via at least one linker L comprising at least one oxygen atom and/or at least one carbon atom.

The phosphorus-based sequestrant(s) may be chosen from inorganic phosphorus-based derivatives, preferably comprising at least 2 phosphorus atoms. More preferentially, the phosphorus-based sequestrant(s) is/are chosen from alkali metal or alkaline-earth metal pyrophosphates, better still from alkali metal pyrophosphates, in particular sodium pyrophosphate (also known as tetrasodium pyrophosphate).

The phosphorus-based sequestrant(s) may be chosen from organic phosphorus-based derivatives, preferably comprising at least 2 phosphorus atoms. More preferentially, the phosphorus-based sequestrant(s) is/are chosen from etidronic acid (also known as 1-hydroxyethane-1,1-diphosphonic acid) and/or alkali metal or alkaline-earth metal, preferably alkali metal, salts thereof, for instance tetrasodium etidronate and disodium etidronate.

Thus, preferably, the phosphorus-based sequestrant(s) is/are chosen from alkali metal pyrophosphates, etidronic acid and/or alkali metal salts thereof, and a mixture of these compounds.

Particularly preferably, the phosphorus-based sequestrant(s) is/are chosen from tetrasodium etidronate, disodium etidronate, etidronic acid, tetrasodium pyrophosphate, and a mixture of these compounds.

According to the present invention, the sequestrants are preferably chosen from diethylenetriaminepentaacetic acid (DTPA) and salts thereof, diethylenediaminetetraacetic acid (EDTA) and salts thereof, ethylenediaminedisuccinic acid (EDDS) and salts thereof, etidronic acid and salts thereof, N,N-dicarboxymethylglutamic acid and salts thereof, N,N-dicarboxymethylglutamic acid and salts thereof (GLDA), and mixtures thereof.

Among the salts of these compounds, the alkali metal salts and especially the sodium or potassium salts are preferred.

When the composition comprises one or more sequestrants, the total content of the sequestrant(s) preferably ranges from 0.001% to 15% by weight, more preferentially from 0.005% to 10% by weight, better still from 0.01% to 8% by weight, even better still from 0.05% to 5% by weight, relative to the total weight of the composition.

### Associative polymer

The composition according to the present invention can comprise one or more associative polymers different from the guar gums according to the invention.

For the purposes of the present invention, the term *"associative polymers"* means water-soluble polymers that are capable, in an aqueous medium, of reversibly associating with each other or with other molecules.

Their chemical structure comprises at least one hydrophilic region and at least one hydrophobic region characterized by at least one C₈-C₃₀ fatty chain.

The associative polymers according to the invention may be of anionic, cationic, amphoteric or nonionic type.

According to a first embodiment, the associative polymer(s) is/are chosen from **anionic associative** polymers. The anionic associative polymers are preferably chosen from:
- (I) those comprising at least one hydrophilic unit and at least one fatty-chain allyl ether unit, more particularly those for which the hydrophilic unit is constituted of an ethylenic unsaturated anionic monomer, more particularly still of a vinylcarboxylic acid and very particularly of an acrylic acid or a methacrylic acid or the mixtures of these, and the fatty-chain allyl ether unit of which corresponds to the monomer of following formula (I):

   CH₂ = C R' CH₂ O Bₙ R (I)

   in which R' denotes H or CH₃, B denotes an ethyleneoxy radical, n is zero or denotes an integer ranging from 1 to 100, R denotes a hydrocarbon-based radical chosen from alkyl, arylalkyl, aryl, alkylaryl and cycloalkyl radicals, comprising from 8 to 30 carbon atoms, preferably 10 to 24, and even more particularly from 12 to 18 carbon atoms. A unit of formula (I) that is more particularly preferred is a unit in which R' denotes H, n is equal to 10 and R denotes a stearyl (C₁₈) radical.

Anionic amphiphilic polymers of this type are described and prepared, according to an emulsion polymerization process, in patent EP-0 216 479.

Among these fatty-chain anionic associative polymers, those that are particularly preferred according to the invention are polymers formed from 20% to 60% by weight of acrylic acid and/or of methacrylic acid, from 5% to 60% by weight of lower alkyl (meth)acrylates, from 2% to 50% by weight of fatty-chain allyl ether of formula (I), and from 0 to 1% by weight of a crosslinking agent which is a well-known copolymerizable polyethylenic unsaturated monomer, for instance diallyl phthalate, allyl (meth)acrylate, divinylbenzene, (poly)ethylene glycol dimethacrylate or methylenebisacrylamide.

Among the latter polymers, preference is very particularly given to crosslinked terpolymers of methacrylic acid, of ethyl acrylate and of polyethylene glycol (10 EO) ether of stearyl alcohol (Steareth 10), especially those sold by the company Allied Colloids under the names Salcare SC 80 and Salcare SC 90, which are aqueous emulsions containing 30% of a crosslinked terpolymer of methacrylic acid, of ethyl acrylate and of steareth-10 allyl ether (40/50/10).
- (II) those comprising at least one hydrophilic unit of unsaturated olefinic carboxylic acid type, and at least one hydrophobic unit of (C₁₀-C₃₀)alkyl ester of unsaturated carboxylic acid type.

These polymers are preferably chosen from those for which the hydrophilic unit of unsaturated olefinic carboxylic acid type corresponds to the monomer of formula (II) below: 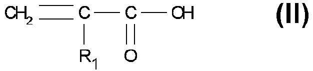 in which R₁ denotes H, CH₃, or C₂H₅, i.e. acrylic acid, methacrylic acid or ethacrylic acid units, and in which the hydrophobic unit of (C₁₀-C₃₀)alkyl ester of unsaturated carboxylic acid type corresponds to the monomer of formula (III) below: 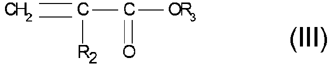 in which R₂ denotes H or CH₃ or C₂H₅ (i.e. acrylate, methacrylate or ethacrylate units) and preferably H (acrylate units) or CH₃ (methacrylate units), R₃ denoting a C₁₀-C₃₀ and preferably C₁₂-C₂₂ alkyl radical.

(C₁₀-C₃₀)Alkyl esters of unsaturated carboxylic acids in accordance with the invention comprise, for example, lauryl acrylate, stearyl acrylate, decyl acrylate, isodecyl acrylate, dodecyl acrylate, and the corresponding methacrylates, lauryl methacrylate, stearyl methacrylate, decyl methacrylate, isodecyl methacrylate and dodecyl methacrylate.

Anionic polymers of this type are described and prepared, for example, according to patents US 3 915 921 and US 4 509 949.

Among the fatty-chain anionic associative polymers of this type, use will more particularly be made of polymers formed from a mixture of monomers comprising:
(i) essentially acrylic acid,
(ii) an ester of formula (III) described above in which R₂ denotes H or CH₃, R₃ denoting an alkyl radical having from 12 to 22 carbon atoms,
(iii) and a crosslinking agent, which is a well-known copolymerizable polyethylenic unsaturated monomer, for instance diallyl phthalate, allyl (meth)acrylate, divinylbenzene, (poly)ethylene glycol dimethacrylate and methylenebisacrylamide.

Among the fatty-chain anionic associative polymers of this type that will be used more particularly are those constituted of from 95% to 60% by weight of acrylic acid (hydrophilic unit), 4% to 40% by weight of C₁₀-C₃₀ alkyl acrylate (hydrophobic unit) and 0 to 6% by weight of crosslinking polymerizable monomer, or else those constituted of from 98% to 96% by weight of acrylic acid (hydrophilic unit), 1% to 4% by weight of C₁₀-C₃₀ alkyl acrylate (hydrophobic unit) and 0.1% to 0.6% by weight of crosslinking polymerizable monomer such as those described previously.

Among the said polymers above, preference is very particularly given, according to the present invention, to the products sold by Goodrich under the trade names Pemulen TR1, Pemulen TR2 and Carbopol 1382, and more preferably still Pemulen TR1, and to the product sold by S.E.P.P.I.C. under the name Coatex SX.
- (III) maleic anhydride/C₃₀-C₃₈ α-olefin/alkyl maleate terpolymers, such as the product (maleic anhydride/C₃₀-C₃₈ α-olefin/isopropyl maleate copolymer) sold under the name Performa V 1608 by Newphase Technologies.
- (IV) acrylic terpolymers comprising:
   (a) approximately 20% to 70% by weight of an α,β-monoethylenically unsaturated carboxylic acid,
   (b) about 20% to 80% by weight of a non-surfactant α,β-monoethylenically unsaturated monomer other than (a),
   (c) about 0.5% to 60% by weight of a nonionic monourethane, which is the product of reaction of a monohydric surfactant with a monoethylenically unsaturated monoisocyanate, such as those described in patent application EP-A-0 173 109 and more particularly the one described in Example 3, namely a methacrylic acid/methyl acrylate/ethoxylated (40 EO) behenyl alcohol dimethyl meta-isopropenyl benzyl isocyanate terpolymer as a 25% aqueous dispersion.
- (V) copolymers comprising among their monomers an α,β-monoethylenically unsaturated carboxylic acid and an ester of an α,β-monoethylenically unsaturated carboxylic acid and of an oxyalkylenated fatty alcohol.

Preferentially, these compounds also comprise as monomer an ester of an α,β-monoethylenically unsaturated carboxylic acid and of a C₁-C₄ alcohol.

An example of a compound of this type that may be mentioned is Aculyn 22 sold by Röhm & Haas, which is a methacrylic acid/ethyl acrylate/oxyalkylenated stearyl methacrylate terpolymer.
- (VI) and mixtures thereof.

According to a second preferred embodiment of the invention, the associative polymer(s) is/are chosen from nonionic associative polymers.

The nonionic associative polymers are preferably chosen from:
(1) celluloses modified with groups including at least one
   fatty chain; Preferably from:
   - hydroxyethylcelluloses modified with groups including at least one fatty chain, such as alkyl, arylalkyl or alkylaryl groups, or mixtures thereof, and in which the alkyl groups are preferably C₈ to C₂₂, preferably such as the cetylhydroxyethylcellulose sold notably under the reference Natrosol Plus Grade 330 CS (C₁₆ alkyls) sold by the company Ashland, or the product Polysurf 67CS sold by the company Ashland,
   - hydroxyethylcelluloses modified with polyalkylene glycol alkylphenyl ether groups, such as the product Amercell Polymer HM-1500 (polyethylene glycol (15) ether of nonyl phenol) sold by the company Amerchol,
   - and mixtures thereof.
(2) copolymers of vinylpyrrolidone and of fatty-chain hydrophobic monomers;
   mention may be made, by way of example, of:
   - the products Antaron V216 or Ganex V216 (vinylpyrrolidone/hexadecene copolymer) sold by the company ISP,
   - the products Antaron V220 or Ganex V220 (vinylpyrrolidone/eicosene copolymer) sold by the company ISP.
(3) copolymers of C₁-C₆ alkyl methacrylates or acrylates and of amphiphilic monomers comprising at least one fatty chain, for instance the oxyethylenated methyl acrylate/stearyl acrylate copolymer sold by the company Goldschmidt under the name Antil 208.
(4) copolymers of hydrophilic methacrylates or acrylates and of hydrophobic monomers comprising at least one fatty chain, for instance the polyethylene glycol methacrylate/lauryl methacrylate copolymer.
(5) polyurethane polyethers comprising in their chain both hydrophilic blocks usually of polyoxyethylenated nature and hydrophobic blocks, which may be aliphatic sequences alone and/or cycloaliphatic and/or aromatic sequences.
(6) polymers comprising an aminoplast ether backbone having at least one fatty chain, such as the Pure Thix compounds sold by Süd-Chemie.
(7) and mixtures thereof.

Preferably, the polyurethane polyethers include at least two hydrocarbon-based lipophilic chains containing from 8 to 30 carbon atoms, separated by a hydrophilic block, the hydrocarbon-based chains possibly being side chains or chains at the end of the hydrophilic block. In particular, it is possible for one or more side chains to be envisaged. In addition, the polymer may include a hydrocarbon-based chain at one end or at both ends of a hydrophilic block.

The polyurethane polyethers may be multiblock, in particular in triblock form. The hydrophobic blocks may be at each end of the chain (for example: triblock copolymer bearing a hydrophilic central block) or distributed both at the ends and in the chain (for example, multiblock copolymer). These same polymers may also be graft polymers or star polymers.

The fatty-chain nonionic polyurethane polyethers may be triblock copolymers, the hydrophilic block of which is a polyoxyethylenated chain including from 50 to 1000 oxyethylene groups. The nonionic polyurethane polyethers include a urethane bond between the hydrophilic blocks, whence the origin of the name.

By extension, the fatty-chain nonionic polyurethane polyethers also include those with hydrophilic blocks bonded to the lipophilic blocks via other chemical bonds.

As examples of fatty-chain nonionic polyurethane polyethers that may be used in the invention, use may also be made of Rheolate 205 containing a urea function, sold by the company Rheox, or Rheolate 208, 204 or 212, and also Acrysol RM 184, Aculyn 44 and Aculyn 46 from the company Röhm & Haas [Aculyn 46 is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44 is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%)].

Mention may also be made of the product Elfacos T210 containing a C₁₂₋₁₄ alkyl chain, and the product Elfacos T212 containing a C₁₈ alkyl chain, from Akzo.

The product DW 1206B from Röhm & Haas containing a C₂₀ alkyl chain and a urethane bond, sold at a solids content of 20% in water, may also be used.

Use may also be made of solutions or dispersions of these polymers, in particular in water or in aqueous-alcoholic medium. Examples of such polymers that may be mentioned are Rheolate 255, Rheolate 278 and Rheolate 244 sold by the company Rheox. Use may also be made of the products DW 1206F and DW 1206J sold by Röhm & Haas.

The polyurethane polyethers that can be used according to the invention are in particular those described in the article by G. Fonnum, J. Bakke and Fk. Hansen - Colloid Polym. Sci., 271, 380-389 (1993).

Particularly preferably, the associative polymer(s) is/are nonionic, and preferably chosen from celluloses modified with groups including at least one fatty chain.

Preferably, the nonionic associative polymer(s) is/are chosen from hydroxyethylcelluloses modified with groups including at least one fatty chain, such as alkyl, arylalkyl or alkylaryl groups, or mixtures thereof, and in which the alkyl groups are preferably C₈-C₂₂, and hydroxyethylcelluloses modified with polyalkylene glycol alkylphenyl ether groups, and mixtures thereof, preferably cetylhydroxyethylcellulose.

According to a third embodiment, the associative polymer(s) is/are chosen from cationic associative polymers.

The term *"cationic polymer"* is intended to mean any polymer comprising cationic groups and/or groups that can be ionized to cationic groups.

Among the cationic associative polymers, mention may be made of:
- (A) cationic associative polyurethanes, which can be represented by general formula (la) below: R-X-(P)n-[L-(Y)m]r-L'-(P')p-X'-R'
   in which:
   **R and R',** which may be identical or different, represent a hydrophobic group or a hydrogen atom;
   **X** and **X',** which may be identical or different, represent a group comprising an amine function optionally bearing a hydrophobic group, or alternatively a group L";
   L, L' and L", which may be identical or different, represent a group derived from a diisocyanate;
   P and P', which may be identical or different, represent a group comprising an amine function optionally bearing a hydrophobic group;
   Y represents a hydrophilic group;
   r is an integer between 1 and 100 inclusive, preferably between 1 and 50 inclusive and in particular between 1 and 25 inclusive;
   n, m and p are each, independently of one another, between 0 and 1000 inclusive;
   the molecule containing at least one protonated or quaternized amine function and at least one hydrophobic group.

Preferably, the only hydrophobic groups are the groups R and R' at the chain ends.

One preferred family of cationic associative polyurethanes is the one corresponding to formula (la) described above, in which:
R and R' both independently represent a hydrophobic group,
X and X' each represent a group L",
n and p are integers that are between 1 and 1000 inclusive, and L, L', L", P, P', Y and m have the meaning indicated above.

Another preferred family of cationic associative polyurethanes is the one corresponding to formula (la) above in which:
- n = p = 0 (the polymers do not include any units derived from a monomer containing an amine function, incorporated into the polymer during the polycondensation),
- the protonated amine functions result from the hydrolysis of excess isocyanate functions, at the chain end, followed by alkylation of the primary amine functions formed with alkylating agents containing a hydrophobic group, i.e. compounds of the type RQ or R'Q, in which R and R' are as defined above and Q denotes a leaving group such as a halide or a sulfate.

Yet another preferred family of cationic associative polyurethanes is the one corresponding to formula (Ia) above in which:
R and R' both independently represent a hydrophobic group,
X and X' both independently represent a group comprising a quaternary amine,
n = p = 0, and
L, L', Y and m have the meaning given above.

The number-average molecular weight (Mn) of the cationic associative polyurethanes is preferably between 400 and 500 000 inclusive, in particular between 1000 and 400 000 inclusive and ideally between 1000 and 300 000 inclusive.

The term "hydrophobic group" is intended to mean a radical or polymer containing a saturated or unsaturated, linear or branched hydrocarbon-based chain, which may contain one or more heteroatoms such as P, O, N or S, or a radical containing a perfluoro or silicone chain. When the hydrophobic group denotes a hydrocarbon-based radical, it includes at least 10 carbon atoms, preferably from 10 to 30 carbon atoms, in particular from 12 to 30 carbon atoms and more preferentially from 18 to 30 carbon atoms.

Preferentially, the hydrocarbon-based group is derived from a monofunctional compound.

By way of example, the hydrophobic group may be derived from a fatty alcohol such as stearyl alcohol, dodecyl alcohol or decyl alcohol. It may also denote a hydrocarbon-based polymer, for instance polybutadiene.

When X and/or X' denote a group comprising a tertiary or quaternary amine, X and/or X' may represent one of the following formulae: 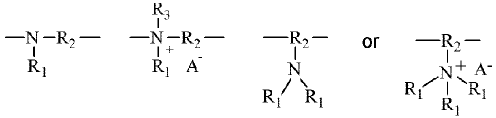 for X 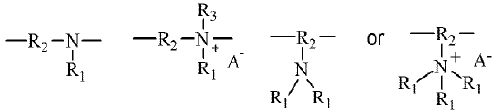 for X' in which:
R2 represents a linear or branched alkylene radical containing from 1 to 20 carbon atoms, optionally comprising a saturated or unsaturated ring, or an arylene radical, one or more of the carbon atoms possibly being replaced with a heteroatom chosen from N, S, O and P;
R1 and R3, which may be identical or different, denote a linear or branched C1-C30 alkyl or alkenyl radical or an aryl radical, at least one of the carbon atoms possibly being replaced with a heteroatom chosen from N, S, O and P;
A⁻ is a physiologically acceptable anionic counterion such as a halide, for instance a chloride or bromide, or a mesylate.

The groups L, L' and L" represent a group of formula: 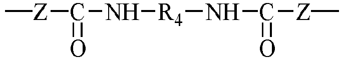 in which:
Z represents -O-, -S- or -NH-; and
R4 represents a linear or branched alkylene radical containing from 1 to 20 carbon atoms, optionally comprising a saturated or unsaturated ring, or an arylene radical, one or more of the carbon atoms possibly being replaced with a heteroatom chosen from N, S, O and P.

The groups P and P' comprising an amine function may represent at least one of the following formulae: 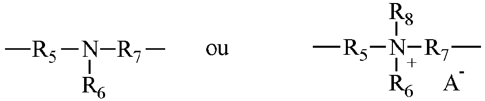 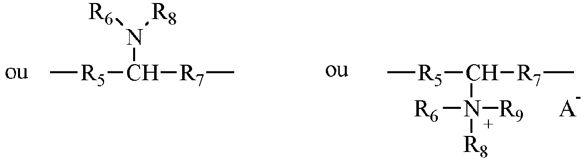 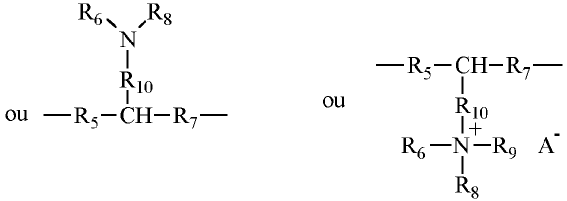 in which:
R5 and R7 have the same meanings as R2 defined above;
R6, R8 and R9 have the same meanings as R1 and R3 defined above;
R10 represents a linear or branched, optionally unsaturated alkylene group possibly containing one or more heteroatoms chosen from N, O, S and P;
and A⁻ is a physiologically acceptable anionic counterion such as a halide, for instance chloride or bromide, or mesylate.

As regards the meaning of Y, the term "hydrophilic group" is intended to mean a polymeric or non-polymeric water-soluble group.

By way of example, when it is not a polymer, mention may be made of ethylene glycol, diethylene glycol and propylene glycol.

When it is a hydrophilic polymer, in accordance with one preferred embodiment, mention may be made, for example, of polyethers, sulfonated polyesters, sulfonated polyamides or a mixture of these polymers. The hydrophilic compound is preferentially a polyether and in particular a poly(ethylene oxide) or poly(propylene oxide).

The cationic associative polyurethanes of formula (la) according to the invention are formed from diisocyanates and from various compounds bearing functions containing labile hydrogen. The functions containing labile hydrogen may be alcohol, primary or secondary amine or thiol functions, giving, after reaction with the diisocyanate functions, polyurethanes, polyureas and polythioureas, respectively. In the present invention, the term "polyurethanes" encompasses these three types of polymer, namely polyurethanes per se, polyureas and polythioureas, and also copolymers thereof.

A first type of compound involved in the preparation of the polyurethane of formula (Ia) is a compound comprising at least one unit bearing an amine function. This compound may be multifunctional, but the compound is preferentially difunctional, that is to say that, according to a preferential embodiment, this compound includes two labile hydrogen atoms borne, for example, by a hydroxyl, primary amine, secondary amine or thiol function. A mixture of multifunctional and difunctional compounds in which the percentage of multifunctional compounds is low may also be used.

As mentioned above, this compound may include more than one unit containing an amine function. In this case, it is a polymer bearing a repetition of the unit containing an amine function.

Compounds of this type may be represented by one of the following formulae:
HZ-(P)ₙ-ZH, or HZ-(P')ₚ-ZH, in which Z, P, P', n and p are as defined above.

Examples that may be mentioned include N-methyldiethanolamine, N-tert-butyldiethanolamine and N-sulfoethyldiethanolamine.

The second compound involved in the preparation of the polyurethane of formula (la) is a diisocyanate corresponding to the formula: O=C=N-R4-N=C=O in which R4 is defined above.

By way of example, mention may be made of methylenediphenyl diisocyanate, methylenecyclohexane diisocyanate, isophorone diisocyanate, toluene diisocyanate, naphthalene diisocyanate, butane diisocyanate and hexane diisocyanate.

A third compound involved in the preparation of the polyurethane of formula (la) is a hydrophobic compound intended to form the terminal hydrophobic groups of the polymer of formula (la).

This compound is formed from a hydrophobic group and a function containing a labile hydrogen, for example a hydroxyl, primary or secondary amine, or thiol function.

By way of example, this compound may be a fatty alcohol such as stearyl alcohol, dodecyl alcohol or decyl alcohol. When this compound includes a polymeric chain, it may be, for example, α-hydroxylated hydrogenated polybutadiene.

The hydrophobic group of the polyurethane of formula (la) may also result from the quaternization reaction of the tertiary amine of the compound comprising at least one tertiary amine unit. Thus, the hydrophobic group is introduced via the quaternizing agent. This quaternizing agent is a compound of the type RQ or R'Q, in which R and R' are as defined above and Q denotes a leaving group such as a halide, a sulfate, etc.

The cationic associative polyurethane may also comprise a hydrophilic block. This block is provided by a fourth type of compound involved in the preparation of the polymer. This compound may be multifunctional. It is preferably difunctional. It is also possible to have a mixture in which the percentage of multifunctional compound is low.

The functions containing labile hydrogen are alcohol, primary or secondary amine or thiol functions. This compound may be a polymer terminated at the chain ends with one of these functions containing labile hydrogen.

By way of example, when it is not a polymer, mention may be made of ethylene glycol, diethylene glycol and propylene glycol.

When it is a hydrophilic polymer, mention may be made, for example, of polyethers, sulfonated polyesters and sulfonated polyamides, or a mixture of these polymers. The hydrophilic compound is preferentially a polyether and in particular a poly(ethylene oxide) or poly(propylene oxide).

The hydrophilic group denoted Y in formula (la) is optional. Specifically, the units containing a quaternary or protonated amine function may suffice to provide the solubility or water-dispersibility required for this type of polymer in an aqueous solution.

Although the presence of a hydrophilic group Y is optional, cationic associative polyurethanes comprising such a group are, however, preferred.
- (B) quaternized cellulose derivatives, and in particular quaternized celluloses modified with groups comprising at least one fatty chain, such as linear or branched alkyl groups, linear or branched arylalkyl groups, or linear or branched alkylaryl groups, preferably linear or branched alkyl groups, these groups comprising at least 8 carbon atoms, in particular from 8 to 30 carbon atoms, better still from 10 to 24, or even from 10 to 14, carbon atoms; or mixtures thereof.

Preferably, mention may be made of quaternized hydroxyethylcelluloses modified with groups comprising at least one fatty chain, such as linear or branched alkyl groups, linear or branched arylalkyl groups, or linear or branched alkylaryl groups, preferably linear or branched alkyl groups, these groups comprising at least 8 carbon atoms, in particular from 8 to 30 carbon atoms, better still from 10 to 24, or even from 10 to 14, carbon atoms; or mixtures thereof.

Preferentially, mention may be made of the hydroxyethylcelluloses of formula (Ib): 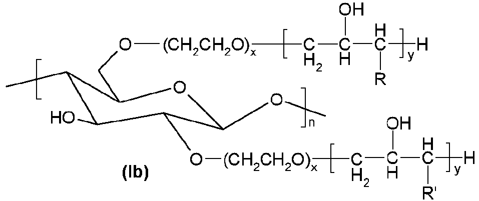 in which:
- R represents an ammonium group RaRbRcN⁺-, Q⁻ in which Ra, Rb and Rc, which may be identical or different, represent a hydrogen atom or a linear or branched C1-C30 alkyl, and Q⁻ represents an anionic counterion such as a halide, for instance a chloride or bromide; preferably an alkyl;
- R' represents an ammonium group R'aR'bR'cN⁺-, Q'⁻ in which R'a, R'b and R'c, which may be identical or different, represent a hydrogen atom or a linear or branched C1-C30 alkyl, and Q'⁻ represents an anionic counterion such as a halide, for instance a chloride or bromide; preferably an alkyl;
   it being understood that at least one of the radicals Ra, Rb, Rc, R'a, R'b and R'c represents a linear or branched C8-C30 alkyl;
- n, x and y, which may be identical or different, represent an integer between 1 and 10 000.

Preferably, in formula (Ib), at least one of the radicals Ra, Rb, Rc, R'a, R'b and R'c represents a linear or branched C8-C30, better still C10-C24, or even C10-C14, alkyl; mention may be made in particular of the dodecyl radical (C12). Preferably, the other radical(s) represent a linear or branched C1-C4 alkyl, in particular methyl.

Preferably, in formula (Ib), only one of the radicals Ra, Rb, Rc, R'a, R'b and R'c represents a linear or branched C8-C30, better still C10-C24, or even C10-C14, alkyl; mention may be made in particular of the dodecyl radical (C12). Preferably, the other radicals represent a linear or branched C1-C4 alkyl, in particular methyl.

Even better still, R may be a group chosen from -N⁺(CH₃)₃, Q'⁻ and -N⁺(C₁₂H₂₅)(CH₃)₂, Q'⁻, preferably an -N⁺(CH₃)₃, Q'⁻ group.

Even better still, R' may be an -N⁺(C₁₂H₂₅)(CH₃)₂, Q'⁻ group.

The aryl radicals preferably denote phenyl, benzyl, naphthyl or anthryl groups.

Mention may notably be made of the polymers having the following INCI names:
- Polyquaternium-24, such as the product Quatrisoft LM 200^{®}, sold by Amerchol/Dow Chemical;
- PG-Hydroxyethylcellulose Cocodimonium Chloride, such as the product Crodacel QM^{®};
- PG-Hydroxyethylcellulose Lauryldimonium Chloride (C12 alkyl), such as the product Crodacel QL^{®}; and
- PG-Hydroxyethylcellulose Stearyldimonium Chloride (C18 alkyl), such as the product Crodacel QS^{®}, sold by Croda.

Mention may also be made of the hydroxyethylcelluloses of formula (Ib) in which R represents trimethylammonium halide and R' represents dimethyldodecylammonium halide; preferentially, R represents trimethylammonium chloride (CH₃)3N⁺-, Cl⁻ and R' represents dimethyldodecylammonium chloride (CH₃)₂(C₁₂H₂₅)N⁺-, Cl⁻. This type of polymer is known under the INCI name Polyquaternium-67; as commercial products, mention may be made of the Softcat Polymer SL^{®} polymers, such as SL-100, SL-60, SL-30 and SL-5, from Amerchol/Dow Chemical.

More particularly, the polymers of formula (Ib) are those of which the viscosity is between 2000 and 3000 cPs inclusive, preferentially between 2700 and 2800 cPs. Typically, Softcat Polymer SL-5 has a viscosity of 2500 cPs, Softcat Polymer SL-30 has a viscosity of 2700 cPs, Softcat Polymer SL-60 has a viscosity of 2700 cPs and Softcat Polymer SL-100 has a viscosity of 2800 cPs.
- (C) cationic polyvinyllactams, in particular those comprising:
   a) at least one monomer of vinyllactam or alkylvinyllactam type;
   b) at least one monomer of structure (Ic) or (IIc) below: 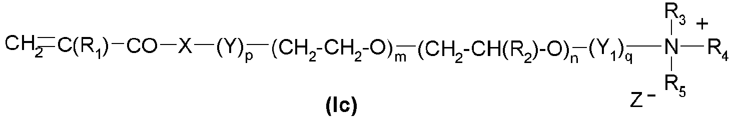 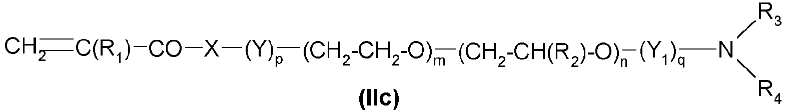 in which:
      - X denotes an oxygen atom or an NR6 radical,
      - R1 and R6 denote, independently of one another, a hydrogen atom or a linear or branched C1-C5 alkyl radical,
      - R2 denotes a linear or branched C1-C4 alkyl radical,
      - R3, R4 and R5 denote, independently of one another, a hydrogen atom, a linear or branched C1-C30 alkyl radical or a radical of formula (Illc):

         -(Y₂)ᵣ-(CH₂-CH(R₇)-O)ₓ-R₈ **(IIIc)**
      - Y, Y1 and Y2 denote, independently of one another, a linear or branched C2-C16 alkylene radical,
      - R7 denotes a hydrogen atom, or a linear or branched C1-C4 alkyl radical or a linear or branched C1-C4 hydroxyalkyl radical,
      - R8 denotes a hydrogen atom or a linear or branched C1-C30 alkyl radical,
      - p, q and r denote, independently of one another, 0 or 1,
      - m and n denote, independently of one another, an integer ranging from 0 to 100 inclusive,
      - x denotes an integer ranging from 1 to 100 inclusive,
      - Z denotes an anionic counterion of an organic or mineral acid, such as a halide, for instance chloride or bromide, or mesylate;
      with the proviso that:
      - at least one of the substituents R3, R4, R5 or R8 denotes a linear or branched C9-C30 alkyl radical,
      - if m and/or n is other than zero, then q is equal to 1,
      - if m = n = 0, then p or q is equal to 0.

The cationic poly(vinyllactam) polymers according to the invention may be crosslinked or non-crosslinked and may also be block polymers.

Preferably, the counterion Z⁻ of the monomers of formula (Ic) is chosen from halide ions, phosphate ions, the methosulfate ion and the tosylate ion.

Preferably, R3, R4 and R5 denote, independently of one another, a hydrogen atom or a linear or branched C1-C30 alkyl radical.

More preferentially, the monomer b) is a monomer of formula (Ic) for which, preferentially, m and n are equal to 0.

The vinyllactam or alkylvinyllactam monomer is preferably a compound of structure (IVc): 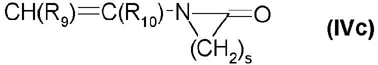 in which:
- s denotes an integer ranging from 3 to 6,
- R9 denotes a hydrogen atom or a linear or branched C1-C5 alkyl radical,
- R10 denotes a hydrogen atom or a linear or branched C1-C5 alkyl radical,
with the proviso that at least one of the radicals R9 and R10 denotes a hydrogen atom.

Even more preferentially, the monomer (IVc) is vinylpyrrolidone.

The cationic poly(vinyllactam) polymers according to the invention may also contain one or more additional monomers, preferably cationic or nonionic monomers.

As compounds that are particularly preferred, mention may be made of the following terpolymers comprising at least:
a) a monomer of formula (IVc),
b) a monomer of formula (Ic) in which p = 1, m = n = q = 0, R3 and R4 denote, independently of one another, a hydrogen atom or a C1-C5 alkyl radical and R5 denotes a linear or branched C9-C24 alkyl radical, and
c) a monomer of formula (IIc) in which p = 1, m = n = q = 0, and R3 and R4 denote, independently of one another, a hydrogen atom or a linear or branched C1-C5 alkyl radical.

Even more preferentially, terpolymers comprising, by weight, 40% to 95% of monomer (a), 0.1% to 55% of monomer (c) and 0.25% to 50% of monomer (b) will be used. Such polymers are in particular described in patent application WO-00/68282.

As cationic poly(vinyllactam) polymers according to the invention, the following are in particular used:
vinylpyrrolidone/dimethylaminopropylmethacrylamide/dodecyldimethylmethacrylamidopro pylammonium tosylate terpolymers,
vinylpyrrolidone/dimethylaminopropylmethacrylamide/cocoyldimethylmethacrylamidopropy lammonium tosylate terpolymers,
vinylpyrrolidone/dimethylaminopropylmethacrylamide/lauryldimethylmethacrylamidopropyl ammonium tosylate or chloride terpolymers.

The vinylpyrrolidone/dimethylaminopropylmethacrylamide/lauryldimethylmethacrylamidopropyl ammonium chloride terpolymer is in particular sold by ISP under the names Styleze W10^{®} and Styleze W20L^{®} (INCI name: Polyquaternium-55).

The weight-average molecular weight (Mw) of the cationic poly(vinyllactam) polymers is preferably between 500 and 20 000 000, more particularly between 200 000 and 2 000 000 and preferentially between 400 000 and 800 000.
- (D) the cationic polymers obtained by polymerization of a monomer mixture comprising one or more vinyl monomers substituted with one or more amino groups, one or more hydrophobic nonionic vinyl monomers, and one or more associative vinyl monomers, as described in patent application WO 2004/024779.

Among these polymers, mention may be made more particularly of the products of polymerization of a monomer mixture comprising:
- a di(C1-C4 alkyl)amino(C1-C6 alkyl) methacrylate,
- one or more C1-C30 alkyl esters of (meth)acrylic acid,
- a polyethoxylated C10-C30 alkyl methacrylate (20-25 mol of ethylene oxide unit),
- a 30/5 polyethylene glycol/polypropylene glycol allyl ether,
- a hydroxy(C2-C6 alkyl) methacrylate, and
- an ethylene glycol dimethacrylate.

Such a polymer is, for example, the compound sold by Lubrizol under the name Carbopol Aqua CC^{®} and which corresponds to the INCI name Polyacrylate-1 Crosspolymer.

According to another embodiment, the associative polymer(s) is/are chosen from **amphoteric associative polymers.**

The term "amphoteric polymers" generally denotes polymers which comprise units K and M distributed randomly in the polymer chain, in which K denotes a unit derived from a monomer including at least one basic nitrogen atom and M denotes a unit derived from an acid monomer including one or more carboxylic or sulfonic groups, or alternatively K and M can denote groups derived from carboxybetaine or sulfobetaine zwitterionic monomers;

K and M can also denote a cationic polymer chain comprising primary, secondary, tertiary or quaternary amine groups, wherein at least one of the amine groups carries a carboxylic or sulfonic group connected via a hydrocarbon-based radical, or alternatively K and M form part of a chain of a polymer comprising an α,β-dicarboxyethylene unit, one of the carboxylic groups of which has been reacted with a polyamine comprising one or more primary or secondary amine groups.

The amphoteric polymers used according to the invention may be chosen for example from polymers derived from polyaspartic acid comprising at least one fatty chain containing from 8 to 30 carbon atoms, such as those:
- described and prepared in patent application EP 0 767 191, the content of which forms an integral part of the present invention. Such polymers are prepared in a known manner, by reaction, in a solvent medium, of polysuccinimide (PSI) with fatty-chain (C₈-C₂₄) amines, in the presence or absence of a basic catalyst such as for example aliphatic tertiary amines, and then amphoterization of the obtained product by reaction with a halogenated organic acid.

Among the fatty-chain C₈-C₂₄ amines that are reacted with PSI, mention may in particular be made of octylamine, nonylamine, decylamine, dodecylamine, tetradecylamine, hexadecylamine, octadecylamine, octadecenylamine, eicosyldecylamine, octynylamine, decenylamine, dodecenylamine, tetradecenylamine, hexadecenylamine, octadecenylamine, and eicosenylamine.

Examples of such polymers are prepared by reaction of PSI with n-laurylamine or n-stearylamine in the presence of N,N-dimethyl-1,3-propanediamine as basic catalyst, followed by amphoterization of the obtained product by reaction with potassium monochloroacetate. These polymers are prepared, in more detail, on pages 13 to 20 (lines 1-4) and in examples 1 to 5 on pages 28 to 34 (lines 1-4) of said patent application EP 0 767 191.
- described and prepared in patent application EP 0 884 344, the content of which forms an integral part of the present invention. Such polymers are prepared by reaction, in a solvent medium, of gaseous ammonia with a C₈-C₂₄ alkyl or alkenyl monomaleate under reduced pressure and at a temperature of 120-140°C for 4 to 6 hours.

The C₈-C₂₄ alkyl or alkenyl radicals may in particular be chosen from the following, linear or branched, radicals: decyl, dodecyl, tetradecyl, hexadecyl, octadecyl, oleyl.

Examples of such polymers include polymers containing aspartic acid units and decyl aspartate units, polymers containing aspartic acid units and dodecyl aspartate units, polymers containing aspartic acid units and cetyl aspartate units, polymers containing aspartic acid units and stearyl aspartate units, and polymers containing aspartic acid units and n-decylaspartamide units, described in examples 1 to 6 of said patent application.
- described and prepared in patent application EP 0 959 094, the content of which forms an integral part of the present invention. Such polymers are prepared by reaction, in a solvent medium, of gaseous ammonia with a polyoxyalkylenated maleic acid monoamide hydrophobically modified with a linear or branched C8-C30 alkyl or alkenyl chain, optionally as a mixture with a maleic acid monoester.

An example of a polymer thus prepared is described in example 2 on page 11 of said patent application.
- described and prepared in patent application EP 0 959 090, the content of which forms an integral part of the present invention. Such hydrophobically modified polymers having a high molecular weight are obtained from maleic acid derivatives and gaseous ammonia and difunctional or polyfunctional amines or alcohols.

Examples of copolymers containing aspartic acid and decyl aspartate units or containing aspartic acid and cetyl aspartate units are given in examples 3 and 5, respectively, of said patent application.
- or else those described and prepared in patent application EP 0 959 091, the content of which forms an integral part of the present invention. Such hydrophobically modified polymers are prepared from maleic acid monoester or monoamide and gaseous ammonia.

Examples of such copolymers are given in examples 1, 2, 3 and 5 of said patent application.

Preferably according to the present invention, the amphoteric polymers are chosen from those comprising at least one non-cyclic cationic unit. More particularly still, preference is given to those prepared from or comprising 1 to 20 mol%, preferably 1.5 to 15 mol% and even more particularly 1.5 to 6 mol% of monomer comprising a fatty chain, relative to the total number of moles of monomers.

Said fatty-chain amphoteric polymers that are preferred according to the invention comprise or are prepared by copolymerizing:
1) at least one monomer of formula (la) or (Ib): 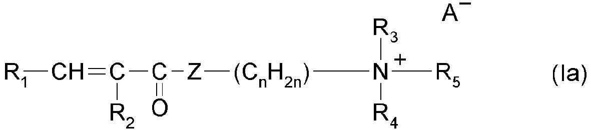 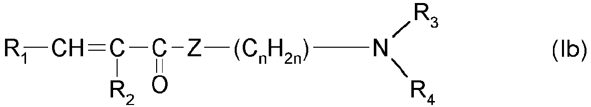 in which R1 and R2, which may be identical or different, represent a hydrogen atom or a methyl radical, R3, R4 and R5, which may be identical or different, represent a linear or branched alkyl radical containing from 1 to 30 carbon atoms,
   Z represents an NH group or an oxygen atom,
   n is an integer from 2 to 5,
   A⁻ is an anion derived from an organic or mineral acid, such as a methosulfate anion or a halide such as chloride or bromide;
2) at least one monomer of formula (II)

   R₆⁻CH=CR₇⁻COOH (II)

   in which R6 and R7, which may be identical or different, represent a hydrogen atom or a methyl radical;
   and
3) at least one monomer of formula (III):

   R₆ - CH=CR₇-COXR₈ (III)

   in which R6 and R7, which may be identical or different, represent a hydrogen atom or a methyl radical, X denotes an oxygen or nitrogen atom and R8 denotes a linear or branched alkyl radical containing from 1 to 30 carbon atoms;
   at least one of the monomers of formula (Ia), (Ib) or (III) comprising at least one fatty chain.

The monomers of formulae (la) and (lb) of the present invention are preferably chosen from the group formed by:
- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide, dimethylaminopropylacrylamide,
these monomers optionally being quaternized, for example with a C1-C4 alkyl halide or a C1-C4 dialkyl sulfate.

More particularly, the monomer of formula (la) is chosen from acrylamidopropyltrimethylammonium chloride and methacrylamidopropyltrimethylammonium chloride.

The monomers of formula (II) of the present invention are preferably chosen from the group formed by acrylic acid, methacrylic acid, crotonic acid and 2-methylcrotonic acid. More particularly, the monomer of formula (II) is acrylic acid.

The monomers of formula (III) of the present invention are preferably chosen from the group formed by C12-C22 and more particularly C16-C18 alkyl acrylates or methacrylates.

The monomers constituting the fatty-chain amphoteric polymers of the invention are preferably already neutralized and/or quaternized.

The ratio of the number of cationic charges/anionic charges is preferably equal to about 1.

The fatty-chain amphoteric polymers according to the invention preferably comprise from 1 to 10 mol% of the monomer comprising a fatty chain (monomer of formula (Ia), (Ib) or (III)), and preferably from 1.5 to 6 mol%.

The weight-average molecular weights of the fatty-chain amphoteric polymers according to the invention may range from 500 to 50 000 000 and are preferably between 10 000 and 5 000 000.

The fatty-chain amphoteric polymers according to the invention may also contain other monomers such as nonionic monomers and in particular such as C1-C4 alkyl acrylates or methacrylates.

Fatty-chain amphoteric polymers according to the invention are described and prepared, for example, in patent application WO 98/44012.

Among the fatty-chain amphoteric polymers according to the invention, preference is given to acrylic acid/(meth)acrylamidopropyltrimethylammonium chloride/stearyl methacrylate terpolymers.

Preferably, the associative polymer(s) is/are chosen from cationic and/or nonionic associative polymers, more preferentially from cationic associative polymers, better still from cationic polymers (B) derived from quaternized cellulose, particularly chosen from the hydroxyethylcelluloses of formula (Ib) and even better still polyquaternium-67.

When the composition comprises one or more associative polymers, the total content of associative polymer(s) preferably ranges from 0.01% to 20% by weight, more preferentially from 0.05% to 10% by weight, better still from 0.075% to 5% by weight, even better still from 0.1% to 3% by weight, relative to the total weight of the composition.

When the composition comprises one or more cationic associative polymers, the total content of cationic associative polymer(s) preferably ranges from 0.01% to 20% by weight, more preferentially from 0.05% to 10% by weight, better still from 0.075% to 5% by weight, even better still from 0.1% to 3% by weight, relative to the total weight of the composition.

### Alkaline agent

The composition according to the present invention may comprise one or more mineral, organic or hybrid alkaline agents.

Preferably, the composition according to the present invention comprises one or more mineral, organic or hybrid alkaline agents.

For the purposes of the present invention, the terms *"alkaline agent"* and *"basifying agent"* are used interchangeably.

The mineral basifying agent(s) is/are preferably chosen from aqueous ammonia, alkali metal carbonates or bicarbonates such as sodium (hydrogen)carbonate and potassium (hydrogen)carbonate, alkali metal or alkaline-earth metal phosphates such as sodium phosphates or potassium phosphates, sodium or potassium hydroxides, and mixtures thereof.

The organic basifying agent(s) is/are preferably chosen from alkanolamines, amino acids, organic amines, oxyethylenated and/or oxypropylenated ethylenediamines, 1,3-diaminopropane, 1,3-diamino-2-propanol, spermine or spermidine and mixtures thereof.

The term "alkanolamine" is intended to mean an organic amine comprising a primary, secondary or tertiary amine function, and one or more linear or branched C₁-C₈ alkyl groups bearing one or more hydroxyl radicals.

Organic amines chosen from alkanolamines such as monoalkanolamines, dialkanolamines or trialkanolamines comprising one to three identical or different C₁-C₄ hydroxyalkyl radicals are in particular suitable for carrying out the invention.

In particular, the alkanolamine(s) is/are chosen from monoethanolamine (MEA), diethanolamine, triethanolamine, monoisopropanolamine, diisopropanolamine, N,N-dimethylethanolamine, 2-amino-2-methyl-1-propanol, triisopropanolamine, 2-amino-2-methyl-1,3-propanediol, 3-amino-1,2-propanediol, 3-dimethylamino-1,2-propanediol, tris(hydroxymethyl)aminomethane and mixtures thereof.

Advantageously, the amino acids are basic amino acids comprising an additional amine function. Such basic amino acids are preferably chosen from histidine, lysine, arginine, ornithine and citrulline.

The organic amine may also be chosen from organic amines of heterocyclic type. Besides histidine that has already been mentioned in the amino acids, mention may in particular be made of pyridine, piperidine, imidazole, triazole, tetrazole and benzimidazole. The organic amine may also be chosen from amino acid dipeptides. As amino acid dipeptides that may be used in the present invention, mention may notably be made of carnosine, anserine and balenine. The organic amine may also be chosen from compounds comprising a guanidine function. As amines of this type other than arginine that may be used in the present invention, mention may especially be made of creatine, creatinine, 1,1-dimethylguanidine, 1,1-diethylguanidine, glycocyamine, metformin, agmatine, n-amidoalanine, 3-guanidinopropionic acid, 4-guanidinobutyric acid and 2-([amino(imino)methyl]amino)ethane-1-sulfonic acid.

Use may be made in particular of guanidine carbonate or monoethanolamine hydrochloride as hybrid compounds.

The alkaline agent(s) that may be used according to the invention is/are preferably chosen from alkanolamines such as monoethanolamine, diethanolamine, triethanolamine; aqueous ammonia, carbonates or bicarbonates such as sodium (hydrogen)carbonate and potassium (hydrogen)carbonate and mixtures thereof, more preferentially from alkanolamines.

When the composition comprises at least one alkaline agent, the total content of the alkaline agent(s) preferably ranges from 0.1% to 40% by weight, more preferentially from 0.5% to 30% by weight, better still from 1% to 20% by weight, even better still from 2% to 10% by weight, relative to the total weight of the composition.

According to one embodiment, the pH of the composition comprising at least one alkaline agent is between 8 and 13; preferably between 9.0 and 12.

The pH of the composition may be adjusted to the desired value by means of acidic or alkaline agent(s) commonly used in the dyeing of keratin fibres, such as those described hereinabove, or alternatively using buffer systems known to those skilled in the art.

According to a particular embodiment, the composition according to the invention comprises:
- 2-(methoxymethyl)benzene-1,4-diamine of formula (I), its addition salts, its solvates and/or the solvates of its salts: 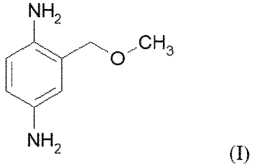
- at least one guar gum;
- at least one fatty substance, the total content of fatty substance being greater than or equal to 30% by weight, relative to the total weight of the composition;
- at least one oxidation coupler;
- at least one surfactant;
- at least one sequestrant; and
- at least one alkaline agent.

### Solvents

The composition according to the invention may also comprise at least one organic solvent.

Examples of organic solvents that may be mentioned include linear or branched C₂ to C₄ alkanols, such as ethanol and isopropanol; polyols and polyol ethers, for instance 2-butoxyethanol, glycerol, propylene glycol, dipropylene glycol, propylene glycol monomethyl ether, diethylene glycol monomethyl ether and monoethyl ether, and also aromatic alcohols or ethers, for instance benzyl alcohol or phenoxyethanol, and mixtures thereof.

The organic solvent(s) may be present in an amount ranging from 0.01% to 30% by weight, preferably ranging from 2% to 25% by weight, relative to the total weight of the composition.

In addition, the composition according to the invention is preferably an aqueous composition. The composition preferably comprises water in an amount of greater than or equal to 5% by weight, preferably greater than or equal to 10% by weight, and better still greater than or equal to 15% by weight, relative to the total weight of the composition.

### Additives

The composition according to the invention may optionally comprise one or more additives, different from the compounds of the invention and among which mention may be made of cationic, anionic, nonionic or amphoteric polymers or mixtures thereof, other than those mentioned hereinabove, antidandruff agents, anti-seborrhoeic agents, agents for preventing hair loss and/or for promoting hair regrowth, vitamins and provitamins including panthenol, sunscreens, mineral or organic pigments, plasticizers, solubilizers, acidifying agents, mineral or organic thickeners, in particular polymeric thickeners other than those described hereinabove, opacifiers or pearlescent agents, antioxidants, hydroxy acids, fragrances, and preservatives.

Of course, those skilled in the art will take care to choose this or these optional additional compound(s) so that the advantageous properties intrinsically associated with the composition according to the invention are not, or not substantially, detrimentally affected by the envisioned addition(s).

The above additives may generally be present in an amount, for each of them, of between 0 and 20% by weight relative to the total weight of the ready-to-use composition.

According to a particular embodiment, the composition according to the invention does not comprise chemical oxidizing agents.

The term "chemical oxidizing agent" means an oxidizing agent other than atmospheric oxygen.

### Process

The present invention also relates to a process for dyeing keratin fibres, preferably the hair, which comprises the application to said keratin fibres of an effective amount of a composition as described previously.

The composition may be applied to wet or dry keratin fibres. On conclusion of the treatment, the keratin fibres are optionally rinsed with water, optionally washed with a shampoo and then rinsed with water, before being dried or left to dry.

According to one embodiment, the composition according to the invention is mixed at the moment of use with a composition comprising at least one chemical oxidizing agent, preferably hydrogen peroxide.

Preferably, the pH of such resulting composition is between 8 and 11, preferably between 9and 10,7.

According to another embodiment, the composition according to the invention comprises at least one oxidizing agent, preferably hydrogen peroxide.

According to this embodiment, at the moment of use, the composition according to the invention results from the mixing of at least two compositions:
a dye composition comprising
   - 2-(methoxymethyl)benzene-1,4-diamine of formula (I), its addition salts, its solvates and/or the solvates of its salts: 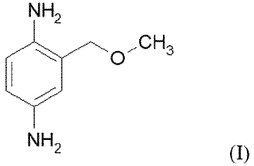
   - at least one guar gum;
   - at least one fatty substance, the total content of fatty substance being greater than or equal to 30% by weight, relative to the total weight of the composition, and
an oxidizing composition comprising one or more chemical oxidizing agents, preferably hydrogen peroxide.

According to one embodiment, the process according to the invention comprises a step of mixing the composition according to the invention with an oxidizing composition comprising at least one chemical oxidizing agent. This mixing step is preferably performed at the moment of use, just before applying the composition resulting from the mixing to the hair.

According to this embodiment, the process for dyeing keratin fibres, preferably the hair, according to the invention, comprises the step of applying, to the keratin fibres, a composition resulting from the mixing, at the moment of use, of at least two compositions:
**a)** a dye composition comprising
   - 2-(methoxymethyl)benzene-1,4-diamine of formula (I), its addition salts, its solvates and/or the solvates of its salts: 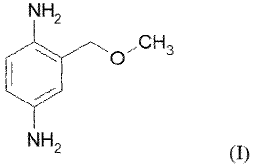
   - at least one guar gum;
   - at least one fatty substance, the total content of fatty substance being greater than or equal to 30% by weight, relative to the total weight of the composition, and
**b)** an oxidizing composition comprising one or more chemical oxidizing agents, preferably hydrogen peroxide.

In a preferred embodiment, the process for dyeing keratin fibres, preferably the hair, according to the invention, comprises the step of applying, to the keratin fibres, a composition resulting from the mixing, at the moment of use, of at least two compositions:
**a)** a dye composition comprising:
   - 2-(methoxymethyl)benzene-1,4-diamine of formula (I), its addition salts, its solvates and/or the solvates of its salts: 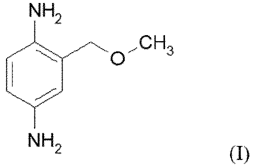
   - at least one guar gum;
   - at least one fatty substance, the total content of fatty substance being greater than or equal to 30% by weight, relative to the total weight of the composition,
   - at least one oxidation coupler;
   - at least one surfactant;
   - at least one sequestrant;
   - at least one alkaline agent; and
**b)** an oxidizing composition comprising one or more chemical oxidizing agents, preferably hydrogen peroxide.

More particularly, the chemical oxidizing agent(s) is/are chosen from hydrogen peroxide, urea peroxide, alkali metal bromates or ferricyanides, peroxygenated salts, for instance persulfates, perborates, peracids and precursors thereof and percarbonates of alkali metals or alkaline-earth metals, and mixtures thereof. The oxidizing agent is preferably chosen from hydrogen peroxide.

The oxidizing composition is preferably an aqueous composition. In particular, it comprises more than 5% by weight of water, preferably more than 10% by weight of water and even more advantageously more than 20% by weight of water.

It may also comprise one or more organic solvents chosen from those listed previously; these solvents more particularly representing, when they are present, from 1% to 40% by weight and preferably from 5% to 30% by weight, relative to the weight of the oxidizing composition.

The oxidizing composition also preferably comprises one or more acidifying agents. Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

The oxidizing composition may additionally comprise fatty substances such as those described hereinabove, preferably chosen from fatty alcohols, liquid hydrocarbons comprising more than 16 carbon atoms and mixtures thereof, surfactants and polymers.

Usually, the pH of the oxidizing composition, when it is aqueous, is less than 7.

Preferably, the oxidizing composition comprises hydrogen peroxide as oxidizing agent, in aqueous solution, the concentration of which ranges, more particularly, from 0.1% to 50%, more particularly between 0.5% and 20% and even more preferentially between 1% and 15% by weight, relative to the weight of the oxidizing composition.

Preferably, at least one of the (dye or oxidizing) compositions is aqueous.

### Kit

Another subject of the invention is a multi-compartment device, preferably comprising two compartments, for dyeing keratin fibres, preferably the hair, at least a first compartment containing the dye composition according to the invention and at least a second compartment containing an oxidizing composition as described above.

The compositions of the device according to the invention are packaged in separate compartments, optionally accompanied by suitable application means which may be identical or different, such as fine brushes, coarse brushes or sponges.

The device mentioned above may also be equipped with a means for dispensing the desired mixture on the hair, for instance the devices described in patent FR 2 586 913.

Finally, the present invention relates to the use of a composition as described above, for dyeing keratin fibres, and in particular the hair.

The following examples serve to illustrate the invention without, however, being limiting in nature.

### Examples

In the examples that follow, all the amounts are given as weight percentage of active material (AM) relative to the total weight of the composition (unless stated otherwise).

### Dyeing composition

Composition **A1** according to the present invention and comparative composition **A2** were prepared using the ingredients of which the contents are indicated in the table below:

**[Table 1]**

| | **A1** (invention) | **A2** (comparative) |
|---|---|---|
| EDTA | 0.2 | 0.2 |
| MONOETHANOLAMINE | 4.44 | 4.44 |
| 2-METHOXYMETHYL-P-PHENYLENEDIAMINE | 0.9 | 0.9 |
| PARAFFINUM LIQUIDUM (liquid petroleum jelly) | 60 | 60 |
| PEG-40 HYDROGENATED CASTOR OIL | 1 | 1 |
| SODIUM LAURYL SULFATE | 1.24 | 1.24 |
| ASCORBIC ACID | 0.12 | 0.12 |
| SODIUM METABISULFITE | 0.22 | 0.22 |
| COCO-GLUCOSIDE | 3 | 3 |
| HYDROXYBENZOMORPHOLINE | 0.9 | 0.9 |
| HYDROXYPROPYL GUAR | 1 | - |
| HYDROXYETHYLCELLULOSE | - | 1 |
| Water | qs 100 | qs 100 |

### Oxidizing composition

The oxidizing composition **B** was prepared from the ingredients of which the contents are indicated in the table below:

**[Table 2]**

| | **B** |
|---|---|
| SODIUM SALICYLATE | 0.035 |
| TOCOPHEROL | 0.1 |
| TETRASODIUM PYROPHOSPHATE | 0.04 |
| HYDROGEN PEROXIDE | 6 |
| CETEARYL ALCOHOL | 6 |
| HEXADIMETHRINE CHLORIDE | 0.15 |
| POLYQUATERNIUM-6 | 0.2 |
| PARAFFINUM LIQUIDUM | 20 |
| TETRASODIUM ETIDRONATE | 0.06 |
| PHOSPHORIC ACID | qs pH 2.2 |
| PEG-4 RAPESEEDAMIDE | 1.2 |
| GLYCERIN | 0.5 |
| STEARETH-20 | 5 |
| Water | qs 100 |

### Dyeing protocol

At the moment of use, the dye compositions **A1** and **A2** are each mixed with the oxidizing composition **B** in a 1+1 weight ratio.

Each of the mixtures is applied to locks of hair containing 90% natural white (NW) hair in a proportion of 3 g of mixture per 1 g of hair.

After a leave-on time of 30 minutes on a hot plate at 27°C, the hair is rinsed, washed with L'Oréal Professionnel Pro Classics universal concentrated shampoo, diluted to 10%, and dried.

### Results

The colouring of the hair is evaluated in the L*a*b* system, using a Konica Minolta CM-3600A spectrocolorimeter (illuminant D65, angle 10°, specular component included) in the CIELab system.

In this system, L* represents the lightness. The lower the value of L*, the darker and more powerful the colouring obtained. The chromaticity is measured by the values a* and b*, a* representing the red/green axis and b* the yellow/blue axis.

The results are given in the table below:

**[Table 3]**

| | L* |
|---|---|
| Process **A1** + **B** (invention) | 25.46 |
| Process **A2** + **B** (comparative) | 27.68 |

The compositions according to the invention lead to a lower value of L*, thus to a better colour strength, compared to the comparative composition.

## Claims

1. Composition comprising:
- 2-(methoxymethyl)benzene-1,4-diamine of formula (I), its addition salts, its solvates and/or the solvates of its salts: 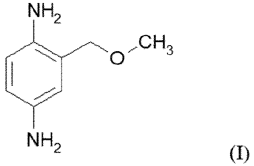
- at least one guar gum,
- at least one fatty substance, the total content of fatty substance being greater than or equal to 30% by weight, relative to the total weight of the composition.

2. Composition according to the preceding claim, wherein the guar gum(s) is/are chosen from nonionic guar gums, more preferentially nonionic guar gums modified with hydroxyalkyl groups.

3. Composition according to any one of the preceding claims, comprising at least one liquid fatty substance, preferably chosen from liquid hydrocarbons containing more than 16 carbon atoms, plant oils, liquid fatty alcohols and liquid fatty esters, silicone oils and mixtures thereof, more preferentially from liquid hydrocarbons containing more than 16 carbon atoms, in particular liquid petroleum jelly.

4. Composition according to any one of the preceding claims, comprising at least one solid fatty substance, preferably chosen from solid fatty acids, solid fatty alcohols, solid esters of fatty acids and/or of fatty alcohols, waxes, ceramides, and mixtures thereof, preferentially from solid fatty acids, solid fatty alcohols and mixtures thereof.

5. Composition according to any one of the preceding claims, wherein the total content of 2-(methoxymethyl)benzene-1,4-diamine of formula (I), of its addition salts, of its solvates and/or of the solvates of its salts ranges from 0.001% to 20% by weight, more preferentially from 0.005% to 15% by weight, better still from 0.01% to 10% by weight, even better still from 0.05% to 5% by weight, better yet from 0.1% to 3% by weight, relative to the total weight of the composition.

6. Composition according to any one of the preceding claims, wherein the total content of guar gums ranges from 0.01% to 20% by weight, more preferentially from 0.05% to 10% by weight, better still from 0.1% to 5% by weight, even better still from 0.5% to 3% by weight, relative to the total weight of the composition.

7. Composition according to any one of the preceding claims, wherein the total content of the fatty substance(s) is greater than or equal to 35% by weight, more preferentially greater than or equal to 40% by weight, better still greater than or equal to 45% by weight, even better still greater than or equal to 50% by weight, relative to the total weight of the composition.

8. Composition according to any one of the preceding claims, wherein the total content of the liquid fatty substance(s) is greater than or equal to 35% by weight, more preferentially greater than or equal to 40% by weight, better still greater than or equal to 45% by weight, even better still greater than or equal to 50% by weight, relative to the total weight of the composition.

9. Composition according to any one of the preceding claims, comprising at least one oxidation coupler preferably chosen from 6-hydroxybenzomorpholine, hydroxyethyl-3,4-methylenedioxyaniline, 2-amino-5-ethylphenol, their addition salts, their solvates and/or the solvates of their salts, and mixtures thereof.

10. Composition according to any one of the preceding claims, comprising at least one surfactant, the surfactant(s) preferably being chosen from anionic surfactants, nonionic surfactants and mixtures thereof, more preferentially chosen from sulfated anionic surfactants, such as alkyl sulfates, ethoxylated C8-C24 fatty alcohols comprising from 1 to 200 ethylene oxide groups, ethoxylated C8-C30 fatty acid esters of sorbitan having from 1 to 30 ethylene oxide units, (C₆-C₂₄ alkyl)polyglycosides, and mixtures thereof.

11. Composition according to any one of the preceding claims, comprising one or more sequestrants.

12. Composition according to any one of the preceding claims, comprising at least one alkaline agent, preferably chosen from alkanolamines such as monoethanolamine, diethanolamine, triethanolamine; aqueous ammonia, carbonates or bicarbonates such as sodium (hydrogen)carbonate and potassium (hydrogen)carbonate and mixtures thereof, more preferentially from alkanolamines.

13. Composition according to any one of the preceding claims, not comprising any chemical oxidizing agent.

14. Composition according to any one of Claims 1 to 13, comprising at least one chemical oxidizing agent, preferably hydrogen peroxide.

15. Process for dyeing keratin fibres, preferably the hair, comprising the application to said keratin fibres of the composition as defined in any one of the preceding claims.

16. Process for dyeing keratin fibres, preferably the hair, according to the preceding claim, **characterized in that** the composition defined according to Claim 14 results from the mixing of at least two compositions:
a) a dye composition comprising
- 2-(methoxymethyl)benzene-1,4-diamine of formula (I), its addition salts, its solvates and/or the solvates of its salts: 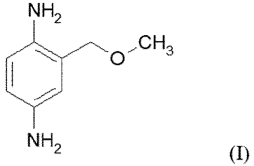
- at least one guar gum,
- at least one fatty substance, the total content of fatty substance being greater than or equal to 30% by weight, relative to the total weight of the composition, and
b) an oxidizing composition comprising one or more chemical oxidizing agents, preferably hydrogen peroxide.

17. Multi-compartment device, preferably comprising two compartments, for dyeing keratin fibres, preferably the hair, at least a first compartment containing a dye composition as defined according to any one of Claims 1 to 13 and at least a second compartment containing an oxidizing composition comprising one or more chemical oxidizing agents, preferably hydrogen peroxide.

18. Use of a composition as defined in any one of Claims 1 to 14, for dyeing keratin fibres, and in particular the hair.

## Patentansprüche

1. Zusammensetzung, umfassend:
- 2-(Methoxymethyl)benzol-1,4-diamin der Formel (I), seine Additionssalze, seine Solvate und/oder die Solvate seiner Salze: 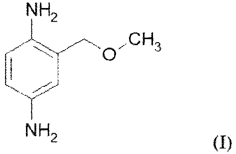
- wenigstens einen Guargummi,
- wenigstens einen Fettstoff, wobei der Gesamtgehalt an Fettstoff größer als oder gleich 30 Gew.-%, bezogen auf das Gesamtgewicht der Zusammensetzung, ist.

2. Zusammensetzung nach dem vorstehenden Anspruch, wobei der/die Guargummi(s) ausgewählt ist/sind aus nichtionischen Guargummis, bevorzugter mit Hydroxyalkylgruppen modifizierten nichtionischen Guargummis.

3. Zusammensetzung nach einem der vorstehenden Ansprüche, umfassend wenigstens einen flüssigen Fettstoff, vorzugsweise ausgewählt aus flüssigen Kohlenwasserstoffen, die mehr als 16 Kohlenwasserstoffatome enthalten, Pflanzenölen, flüssigen Fettalkoholen und flüssigen Fettestern, Siliconölen und Gemischen davon, bevorzugter aus flüssigen Kohlenwasserstoffen, die mehr als 16 Kohlenstoffatome enthalten, insbesondere flüssiger Vaseline.

4. Zusammensetzung nach einem der vorstehenden Ansprüche, umfassend wenigstens einen festen Fettstoff, vorzugsweise ausgewählt aus festen Fettsäuren, festen Fettalkoholen, festen Estern von Fettsäuren und/oder von Fettalkoholen, Wachsen, Ceramiden und Gemischen davon, vorzugsweise aus festen Fettsäuren, festen Fettalkoholen und Gemischen davon.

5. Zusammensetzung nach einem der vorstehenden Ansprüche, wobei der Gesamtgehalt an 2-(Methoxymethyl)benzol-1,4-diamin der Formel (I), seinen Additionssalzen, seinen Solvaten und/oder den Solvaten seiner Salze in dem Bereich von 0,001 Gew.-% bis 20 Gew.-%, bevorzugter von 0,005 Gew.-% bis 15 Gew.-%, noch besser von 0,01 Gew.-% bis 10 Gew.-%, sogar noch besser von 0,05 Gew.-% bis 5 Gew.-%, besser noch von 0,1 Gew.-% bis 3 Gew.-%, bezogen auf das Gesamtgewicht der Zusammensetzung, liegt.

6. Zusammensetzung nach einem der vorstehenden Ansprüche, wobei der Gesamtgehalt an Guargummis in dem Bereich von 0,01 bis 20 Gew.-%, bevorzugter von 0,05 bis 10 Gew.-%, noch besser von 0,1 bis 5 Gew.-%, sogar noch besser von 0,5 bis 3 Gew.-%, bezogen auf das Gesamtgewicht der Zusammensetzung, liegt.

7. Zusammensetzung nach einem der vorstehenden Ansprüche, wobei der Gesamtgehalt an den Fettstoff(en) größer als oder gleich 35 Gew.-%, bevorzugter größer oder gleich 40 Gew.-%, noch besser größer oder gleich 45 Gew.-%, sogar noch besser größer oder gleich 50 Gew.-%, bezogen auf das Gesamtgewicht der Zusammensetzung, ist.

8. Zusammensetzung nach einem der vorstehenden Ansprüche, wobei der Gesamtgehalt an den flüssigen Fettstoff (en) größer als oder gleich 35 Gew.-%, bevorzugter größer oder gleich 40 Gew.-%, noch besser größer oder gleich 45 Gew.-%, sogar noch besser größer oder gleich 50 Gew.-%, bezogen auf das Gesamtgewicht der Zusammensetzung, ist.

9. Zusammensetzung nach einem der vorstehenden Ansprüche, umfassend wenigstens einen Oxidationskuppler, vorzugsweise ausgewählt aus 6-Hydroxybenzomorpholin, Hydroxyethyl-3,4-methylendioxyanilin, 2-Amino-5-ethylphenol, ihren Additionssalzen, ihren Solvaten und/oder den Solvaten ihrer Salze, und Gemischen davon.

10. Zusammensetzung nach einem der vorstehenden Ansprüche, umfassend wenigstens ein Tensid, wobei die Tensid(e) vorzugsweise ausgewählt sind aus anionischen Tensiden, nichtionischen Tensiden und Gemischen davon, bevorzugter ausgewählt aus sulfatierten anionischen Tensiden, wie z.B. Alkylsulfaten, ethoxylierten C8-C24-Fettalkoholen, die von 1 bis 200 Ethylenoxidgruppen umfassen, ethoxylierten C8-C30-Fettsäureestern von Sorbitan mit 1 bis 30 Ethylenoxideinheiten, (C₆-C₂₄-Alkyl)polyglycosiden und Gemischen davon.

11. Zusammensetzung nach einem der vorstehenden Ansprüche, umfassend ein oder mehrere Sequestriermittel.

12. Zusammensetzung nach einem der vorstehenden Ansprüche, umfassend wenigstens ein alkalisches Mittel, vorzugsweise ausgewählt aus Alkanolaminen, wie z.B. Monoethanolamin, Diethanolamin, Triethanolamin; wässrigem Ammoniak, Carbonaten oder Bicarbonaten, wie z.B. Natrium(hydrogen)carbonat und Kalium(hydrogen)carbonat, und Gemischen davon, bevorzugter aus Alkanolaminen.

13. Zusammensetzung nach einem der vorstehenden Ansprüche, die kein chemisches Oxidationsmittel umfasst.

14. Zusammensetzung nach einem der Ansprüche 1 bis 13, umfassend wenigstens ein chemisches Oxidationsmittel, vorzugsweise Wasserstoffperoxid.

15. Verfahren zum Färben von Keratinfasern, vorzugsweise dem Haar, umfassend das Aufbringen der Zusammensetzung nach einem der vorstehenden Ansprüche auf die Keratinfasern.

16. Verfahren zum Färben von Keratinfasern, vorzugsweise dem Haar, nach dem vorstehenden Anspruch, wobei die Zusammensetzung nach Anspruch 14 durch das Mischen von wenigstens zwei Zusammensetzungen entsteht:
a) einer Farbstoffzusammensetzung, umfassend
- 2-(Methoxymethyl)benzol-1,4-diamin der Formel (I), seine Additionssalze, seine Solvate und/oder die Solvate seiner Salze: 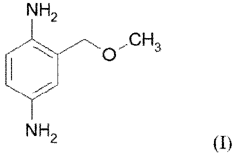
- wenigstens einen Guargummi,
- wenigstens einen Fettstoff, wobei der Gesamtgehalt an Fettstoff größer als oder gleich 30 Gew.-%, bezogen auf das Gesamtgewicht der Zusammensetzung, ist, und
b) einer oxidierenden Zusammensetzung, umfassend ein oder mehrere chemische Oxidationsmittel, vorzugsweise Wasserstoffperoxid.

17. Mehrkompartimentvorrichtung, vorzugsweise umfassend zwei Kompartimente, zum Färben von Keratinfasern, vorzugsweise dem Haar, wobei wenigstens ein erstes Kompartiment eine Farbstoffzusammensetzung nach einem der Ansprüche 1 bis 13 enthält und wenigstens ein zweites Kompartiment eine oxidierende Zusammensetzung, die ein oder mehrere chemische Oxidationsmittel, vorzugsweise Wasserstoffperoxid, umfasst, enthält.

18. Verwendung einer Zusammensetzung nach einem der Ansprüche 1 bis 14 zum Färben von Keratinfasern und insbesondere dem Haar.

## Revendications

1. Composition comprenant :
- de la 2-(méthoxyméthyl)benzène-1,4-diamine de formule (I), ses sels d'addition, ses solvates et/ou les solvates de ses sels : 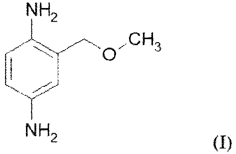
- au moins une gomme de guar,
- au moins une substance grasse, la teneur totale de la substance grasse étant supérieure ou égale à 30 % en poids, par rapport au poids total de la composition.

2. Composition selon la revendication précédente, dans laquelle la ou les gommes de guar sont choisies parmi les gommes de guar non ioniques, plus préférentiellement les gommes de guar non ioniques modifiées par des groupes hydroxyalkyle.

3. Composition selon l'une quelconque des revendications précédentes, comprenant au moins une substance grasse liquide, de préférence choisie parmi les hydrocarbures liquides contenant plus de 16 atomes de carbone, les huiles végétales, les alcools gras liquides et les esters gras liquides, les huiles de silicone et les mélanges de ceux-ci, plus préférentiellement parmi les hydrocarbures liquides contenant plus de 16 atomes de carbone, en particulier la gelée de pétrole liquide.

4. Composition selon l'une quelconque des revendications précédentes, comprenant au moins une substance grasse solide, de préférence choisie parmi les acides gras solides, les alcools gras solides, les esters solides d'acides gras et/ou d'alcools gras, les cires, les céramides et les mélanges de ceux-ci, préférentiellement parmi les acides gras solides, les alcools gras solides et les mélanges de ceux-ci.

5. Composition selon l'une quelconque des revendications précédentes, dans laquelle la teneur totale de la 2-(méthoxyméthyl)benzène-1,4-diamine de formule (I), de ses sels d'addition, de ses solvates et/ou des solvates de ses sels va de 0,001 % à 20 % en poids, plus préférentiellement de 0,005 % à 15 % en poids, mieux encore de 0,01 % à 10 % en poids, même encore mieux de 0,05 % à 5 % en poids, et encore mieux de 0,1 % à 3 % en poids, par rapport au poids total de la composition.

6. Composition selon l'une quelconque des revendications précédentes, dans laquelle la teneur totale des gommes de guar va de 0,01 % à 20 % en poids, plus préférentiellement de 0,05 % à 10 % en poids, mieux encore de 0,1 % à 5 % en poids, même encore mieux de 0,5 % à 3 % en poids, par rapport au poids total de la composition.

7. Composition selon l'une quelconque des revendications précédentes, dans laquelle la teneur totale de la ou les substances grasses est supérieure ou égale à 35 % en poids, plus préférentiellement supérieure ou égale à 40 % en poids, mieux encore supérieure ou égale à 45 % en poids, même encore mieux supérieure ou égale à 50 % en poids, par rapport au poids total de la composition.

8. Composition selon l'une quelconque des revendications précédentes, dans laquelle la teneur totale de la ou les substances grasses liquides est supérieure ou égale à 35 % en poids, plus préférentiellement supérieure ou égale à 40 % en poids, mieux encore supérieure ou égale à 45 % en poids, même encore mieux supérieure ou égale à 50 % en poids, par rapport au poids total de la composition.

9. Composition selon l'une quelconque des revendications précédentes, comprenant au moins un coupleur d'oxydation de préférence choisi parmi la 6-hydroxybenzomorpholine, l'hydroxyéthyl-3,4-méthylènedioxyaniline, le 2-amino-5-éthylphénol, leurs sels d'addition, leurs solvates et/ou les solvates de leurs sels et les mélanges de ceux-ci.

10. Composition selon l'une quelconque des revendications précédentes, comprenant au moins un tensioactif, le ou les tensioactifs étant de préférence choisis parmi les tensioactifs anioniques, les tensioactifs non ioniques et les mélanges de ceux-ci, plus préférentiellement choisis parmi les tensioactifs anioniques sulfatés, tels que les alkylsulfates, les alcools gras en C8-C24 éthoxylés comprenant de 1 à 200 groupes oxyde d'éthylène, les esters d'acides gras en C8-C30 et de sorbitane éthoxylés ayant de 1 à 30 unités oxyde d'éthylène, les (alkyl en C₆-C₂₄)polyglycosides et les mélanges de ceux-ci.

11. Composition selon l'une quelconque des revendications précédentes, comprenant un ou plusieurs séquestrants.

12. Composition selon l'une quelconque des revendications précédentes, comprenant au moins un agent alcalin, de préférence choisi parmi les alcanolamines telles que la monoéthanolamine, la diéthanolamine, la triéthanolamine ; l'ammoniaque, les carbonates ou les bicarbonates aqueux tels que le carbonate ou hydrogénocarbonate de sodium, et le carbonate ou hydrogénocarbonate de potassium et les mélanges de ceux-ci, plus préférentiellement parmi les alcanolamines.

13. Composition selon l'une quelconque des revendications précédentes, ne comprenant aucun agent oxydant chimique.

14. Composition selon l'une quelconque des revendications 1 à 13, comprenant au moins un agent oxydant chimique, de préférence du peroxyde d'hydrogène.

15. Procédé pour la coloration de fibres kératiniques, de préférence des cheveux, comprenant l'application sur lesdites fibres kératiniques de la composition telle que définie dans l'une quelconque des revendications précédentes.

16. Procédé pour la coloration de fibres kératiniques, de préférence des cheveux, selon la revendication précédente, **caractérisé en ce que** la composition définie selon la revendication 14 résulte du mélange d'au moins deux compositions :
a) une composition de colorant comprenant
- de la 2-(méthoxyméthyl)benzène-1,4-diamine de formule (I), ses sels d'addition, ses solvates et/ou les solvates de ses sels : 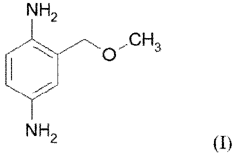
- au moins une gomme de guar,
- au moins une substance grasse, la teneur totale de la substance grasse étant supérieure ou égale à 30 % en poids, par rapport au poids total de la composition, et
b) une composition oxydante comprenant un ou plusieurs agents oxydants chimiques, de préférence du peroxyde d'hydrogène.

17. Dispositif à plusieurs compartiments, de préférence comprenant deux compartiments, pour la coloration de fibres kératiniques, de préférence des cheveux, au moins un premier compartiment contenant une composition de colorant telle que définie selon l'une quelconque des revendications 1 à 13 et au moins un second compartiment contenant une composition oxydante comprenant un ou plusieurs agents oxydants chimiques, de préférence du peroxyde d'hydrogène.

18. Utilisation d'une composition telle que définie dans l'une quelconque des revendications 1 à 14, pour la coloration de fibres kératiniques et en particulier des cheveux.
